# FASCICULE DE BREVET EUROPEEN

(11) **EP 1 957 978 B1**
(45) Date de publication et mention de la délivrance du brevet: **30.12.2015**
(21) Numéro de dépôt: 06819249.1
(22) Date de dépôt: 03.11.2006
(51) Int. Cl.: G01N 33/538, G01N 33/543, G01N 33/555, G01N 33/80

(54) **PROCEDE MAGNETIQUE D'IMMUNODIAGNOSTIC POUR LA MISE EN EVIDENCE DE COMPLEXE ANTICORPS/ANTIGENE, EN PARTICULIER DE GROUPE SANGUIN**
MAGNETISCH-IMMUNDIAGNOSTISCHES VERFAHREN ZUR DEMONSTRATION VON ANTIKÖRPER/ANTIGEN-KOMPLEXEN, VOR ALLEM VON BLUTGRUPPEN
MAGNETIC IMMUNODIAGNOSTIC METHOD FOR THE DEMONSTRATION OF ANTIBODY/ANTIGEN COMPLEXES ESPECIALLY OF BLOOD GROUPS

(30) Priorité: 03.11.2005 FR 0511207
(43) Date de publication de la demande: 20.08.2008
(73) Titulaire: Diagast, 59120 Loos (FR)
(72) Inventeur: BARBREAU, Yves, F-59420 Mouvaux (FR); BOULET, Olivier, F-62113 Sailly Labourse (FR); BOULET, Arnaud, F-62000 Arras (FR); DELANOE, Alexis, F-59110 La Madeleine (FR); FAUCONNIER, Laurence, F-59491 Villeneuve D'ascq (FR); HERBERT, Fabien, F-59000 Lille (FR); PELOSIN, Jean-Marc, F-59130 Lambersart (FR); SOUFFLET, Laurent, F-59230 Saint Amand Les Eaux (FR)
(74) Mandataire: Regimbeau
(86) Numéro de dépôt international: PCT/EP2006/068085
(87) Numéro de publication internationale: WO 2007/051844

(56) Documents cités:
- EP-A- 0 230 768
- EP-A- 0 297 290
- EP-B- 0 351 857
- DE-A1- 4 228 395
- US-A- 4 560 647
- US-A- 5 770 388
- US-A1- 5 318 914
- US-B1- 6 303 390

## Description

La présente invention concerne un procédé magnétique d'immunodiagnostic pour la mise en évidence de complexe anticorps/antigène. Un tel procédé a pour application la recherche et/ou l'identification d'anticorps ou d'antigène, de préférence d'anticorps anti-antigène ou d'antigène de groupe sanguin, ce procédé mettant en oeuvre une suspension de particules magnétiques revêtues d'antigène pouvant être porté par des cellules telles que des érythrocytes. L'invention comprend également un dispositif et un kit permettant de réaliser un tel procédé.

La transfusion sanguine consiste de nos jours à administrer par voie intraveineuse des préparations de concentré de globules rouges (concentrés globulaires) obtenues à partir de sang de donneurs.

Lors d'une transfusion sanguine, le risque premier est lié à la possibilité de réunir dans l'organisme du receveur (la personne transfusée) un anticorps et son antigène érythrocytaire. On trouve en effet à la surface des érythrocytes, appelés encore globules rouges ou hématies, des antigènes membranaires érythrocytaires, notamment des antigènes de groupe (ou système) sanguin, capables d'être reconnus par le système immunitaire et de déclencher une réponse immune.

Les globules rouges du donneur sont dits compatibles avec le sang du receveur si le receveur ne présente pas d'anticorps circulants dirigés contre un antigène érythrocytaire du donneur.

Parmi l'ensemble des variants antigéniques d'un antigène membranaire érythrocytaire constituant les groupes sanguins, plus de vingt systèmes antigéniques érythrocytaires chez l'Homme ont été à ce jour identifiés : système ABO avec les antigènes A ou B, système Rhésus avec l'antigène D, E ou e et C ou c, système Kell avec l'antigène K ou k, Duffy (Fya, Fyb), Kidd (Jka, Jkb) ou encore d'autres systèmes moins fréquemment recherchés en pratique existant aussi tels que MNS, Lewis, etc.. Les individus présentant une même association d'antigènes érythrocytaires appartiennent à un même groupe sanguin érythrocytaire. Les groupes sanguins sont d'autant plus complexes et nombreux que l'on utilise plusieurs systèmes antigéniques.

En dehors de situation pathologique, dans le cas par exemple de maladie auto-immune, le sérum d'un individu peut contenir deux types d'anticorps dirigés contre des antigènes érythrocytaires :
- (i) les anticorps dits réguliers et dirigés contre les antigènes du système ABO (par exemple anticorps anti-A chez l'individu de groupe B). Il s'agit d'immunoglobulines de type IgM qui sont capables d'agglutiner les globules rouges in vitro. Ce phénomène est mis à profit pour déterminer le groupe ABO d'un individu par les épreuves de Beth-Vincent et Simonin, l'épreuve de Beth-Vincent permettant de déterminer les antigènes portés par les globules rouges de l'individu (phénotype antigénique) et l'épreuve de Simonin permettant de réaliser l'étude complémentaire, c'est-à-dire de déterminer les anticorps anti-A et/ou anti-B circulants présents dans le sérum de l'individu.

Dans l'épreuve de Beth-Vincent, les globules rouges de l'individu sont mis en présence de sérums tests, ou anticorps tests, possédant chacun un type d'anticorps précis, dirigé contre un antigène du système ABO. Il s'agit donc d'un test d'agglutination des globules rouges avec des sérums tests.

Dans l'épreuve de Simonin, appelée encore contre-épreuve, le sérum de l'individu, contenant ses anticorps circulants, est mis en présence de globules rouges tests, ou érythrocytes tests, appartenant chacun à un groupe antigénique précis du système ABO. Il s'agit donc d'un test d'agglutination du sérum avec des globules rouges tests :
- (ii) les anticorps dits irréguliers (ou immuns) dont la présence dans le sérum ou plasma est facultative et qui sont dirigés contre des antigènes des systèmes non ABO. Il s'agit le plus souvent d'IgG, apparaissant à l'occasion d'une stimulation antigénique par des globules rouges étrangers, par exemple suite à une immunisation contre un ou plusieurs antigènes lors d'une transfusion sanguine ou encore lors d'une grossesse par réaction immunitaire maternelle dirigée contre les antigènes érythrocytaires foetaux n'appartenant pas au groupe sanguin maternel, notamment lors de l'accouchement.

La recherche de ces anticorps dits irréguliers est appelée Recherche d'Agglutinine Irrégulière (RAI). Ce test est utilisé pour détecter la présence ou non dans le sang d'un individu d'IgG dirigées contre divers antigènes érythrocytaires. Pour cela, on cherche à mettre en évidence la fixation de ces IgG sur des globules rouges tests dont les antigènes sont connus. On procède en parallèle sur de nombreux types de globules rouges, la comparaison des résultats permettant d'identifier l'IgG ou les IgG présentes.

Le risque est plus important pour les antigènes les plus immunogènes comme le rhésus D mais aussi les autres rhésus (E>c>e>C), le Kell (K), le Duffy (Fy a, Fy b), le Kidd (Jka, Jk b), etc..

En pratique, on ne peut tenir compte de tous ces antigènes pour réaliser une transfusion faute de quoi on ne disposerait jamais du bon groupe sanguin au bon moment, d'autant moins que certaines associations antigéniques sont très rares. La transfusion standard ne tient compte que du groupe dans le système ABO plus le Rhésus D (Rh+ ou Rh-). Dans les situations à risque d'apparition d'une agglutinine irrégulière, on tient compte d'un certain nombre d'autres systèmes, notamment les Rhésus C et E et le Kell, voire encore d'autres systèmes. Il s'agira alors pour ces situations à risques de respecter la compatibilité du groupe sanguin du donneur avec celui du sang du receveur en tenant compte de la présence ou du risque d'apparition de ces agglutinines irrégulières.

Ainsi, chez un patient receveur porteur d'anticorps irréguliers anti-érythrocytaires ou dans une situation à risque comme notamment chez les patients polytransfusés ne possédant pas d'anticorps irréguliers anti-érythrocytaires et chez la femme enceinte, il est indispensable de sélectionner les unités de concentrés érythrocytaires qui vont lui être transfusées de telle sorte que les globules rouges du donneur devront être dépourvus des antigènes contre lesquels les anticorps du receveur sont dirigés ou susceptibles d'apparaître. Cette épreuve de compatibilité est obligatoire chez ces patients et de manière préventive chez tous les receveurs avant l'administration des concentrés érythrocytaires en effectuant une épreuve de compatibilité directe avec les hématies du donneur en présence du sérum ou plasma du receveur. Aucune réaction d'agglutination et/ou de lyse dans les techniques utilisées pour la RAI ne doit être observée.

En pratique clinique transfusionnelle, le phénotypage érythrocytaire, qui correspond à la recherche et à l'identification des antigènes de groupe sanguin à la surface des hématies (à l'exception du système particulier ABO où l'on recherche en outre la présence des anticorps réguliers correspondants), concerne aussi bien le receveur que le donneur.

A l'échelon du receveur et du donneur, trois niveaux de phénotype érythrocytaire existent afin de mettre à disposition du receveur des concentrés érythrocytaires compatibles en fonction des situations à risque :
- la détermination du phénotype groupage ABO (ou groupage ABO) et Rhésus standard (présence ou absence de l'antigène D) ;
- la détermination du phénotype Rhésus Kell (présence ou absence des antigènes C, E, c, e et K) ; et
- la détermination du phénotype étendu (ou élargi) (présence ou absence des antigènes du système Duffy, Fy a et Fy b, du Système Kidd, Jk a et Jk b, et du système MNSs (antigènes S et s), d'autres antigènes pouvant être en outre recherchés selon la nature du risque et/ou de l'anticorps irrégulier mis en évidence dans le sérum du receveur.

Les techniques habituellement utilisées, mises en oeuvre pour rechercher et identifier la présence ou l'absence d'antigène de groupe sanguin à la surface d'érythrocyte de donneur et/ou de receveur, ou visant à rechercher et identifier la présence ou l'absence d'anticorps anti-antigène de groupe sanguin, régulier (pour le groupage ABO) ou irrégulier dans le cadre de la RAI dans le sérum ou plasma de donneur et/ou de receveur, sont bien connues de l'homme de l'art et ne seront ici pas décrites.

Pour le phénotypage, elles consistent en général à rechercher à l'aide de sérums-tests contenant les anticorps appropriés la présence ou l'absence de l'antigène recherché. De préférence, ces anticorps contenus dans ces sérums tests sont de nature agglutinante (IgM ou IgA), ce qui permet d'obtenir une agglutination totale ou partielle des érythrocytes à phénotyper lorsque ces derniers portent l'antigène correspondant à l'anticorps présent dans le sérum test. Néanmoins, il est possible d'utiliser des anticorps tests non agglutinants (de type IgG), l'agglutination étant provoquée au moyen d'une anti-immunoglobuline et visible notamment après une étape de centrifugation et remise en suspension du culot obtenu (technique dite de Coombs indirect). Il est également possible d'utiliser des anticorps tests non agglutinants où la présence de ces anticorps tests fixés sur le globule rouge est visualisée au moyen d'une anti-immunoglobuline fixée sur une phase solide (technique dite d'immuno-adhérence). La lecture du résultat pourra être réalisée à l'oeil ou au moyen de lecteur approprié.

Pour la recherche et l'identification dans l'échantillon de sérum ou plasma du patient à tester d'anticorps anti-antigènes de groupe sanguin, réguliers pour le groupage ABO ou irréguliers dans le cadre de la RAI, on met en général en présence du sérum ou du plasma du patient avec des érythrocytes-tests (appelés encore hématies-, ou globules rouges-tests, d'antigénicité connue dans un certain nombre de systèmes de groupes sanguins (ABO, Rhésus, Kell, Duffy, Kidd, MNSs, ...). Pour la RAI, pour laquelle les anticorps susceptibles d'être présents sont plutôt de type non agglutinant, les techniques utilisées sont de type Coombs indirect par agglutination au moyen d'une anti-immunoglobuline ou par immunoadhérence sur une phase solide revêtue d'une anti-immunoglobuline.

Pour la RAI, on utilise dans une première étape un panel de globules rouges dit de dépistage (deux ou trois globules rouges de groupe différent choisis de façon à comporter le maximum d'antigènes) permettant de détecter (mais pas d'identifier) la présence ou l'absence d'anticorps irréguliers. Lorsque le dépistage est positif, on réalise alors l'identification de la spécificité du ou des anticorps irréguliers présents au moyen d'au moins un panel de globules rouges dit d'identification comportant en général 10 globules rouges différents phénotypés dans la grande majorité des systèmes de groupes sanguins connus.

Il existe un grande nombre de variantes des techniques utilisées pour le phénotypage ou la RAI dans le domaine de la transfusion sanguine, ces techniques pouvant être manuelles, sur plaque d'opaline, en tube ou en cupule de microplaque, ou complètement automatisées à l'aide de robot distributeur d'échantillon et de réactif, agitateur, incubateur et lecteur automatique dont les programmes sont adaptés aux techniques mises en oeuvre.

Parmi les techniques utilisées, on peut citer les techniques pour lesquelles la présence d'anticorps anti-antigène de groupe sanguin ou d'antigène de groupe sanguin est basée sur la mise en évidence d'une agglutination de globules rouges après centrifugation mettant en oeuvre une mini colonne de filtration transparente (gel type « sephadex® » ou microbilles) dont l'évasement à l'extrémité supérieure sert de chambre d'incubation et dont le seuil de coupure choisi pour la colonne empêche les hématies agglutinées après centrifugation de traverser la colonne (voir notamment le document brevet EP 0 194 212 ou brevet EP 0 755 719).

On peut également citer les techniques pour lesquelles le phénotypage ou la RAI est basée sur la mise en évidence de globules rouges sensibilisés avec un anticorps après centrifugation puis immunoadhérence mettant en oeuvre une barrière de séparation constituée d'un gel ou d'un liquide dont la densité est choisie de telle manière que seuls les globules rouges peuvent traverser cette barrière lors d'une centrifugation, le container à réaction étant revêtu dans sa partie inférieure d'une anti-immunoglobuline afin de piéger les globules rouges sensibilisés et donner une image caractéristique. Parmi ces techniques, on peut citer le document brevet EP 0 058 780 qui décrit un procédé de phénotypage sanguin dans lequel le mélange réactionnel est centrifugé au travers d'un coussin de densité supérieure (de type solution d'albumine bovine ou de polyvinyl pyrrolidone), ce qui présente l'avantage de supprimer l'étape de lavage des hématies sensibilisées. On peut également citer le document brevet WO 98/02752 qui décrit un procédé général pour déterminer la présence d'un antigène sanguin présent sur des érythrocytes ou d'un anticorps fixant un tel antigène. Dans ce procédé, les érythrocytes sensibilisés ou non, sont séparés des anticorps non fixés par centrifugation grâce à un milieu de séparation dont la densité est supérieure à celle du liquide contenant les anticorps, mais inférieure à celle des érythrocytes, les érythrocytes sensibilisés étant séparés des érythrocytes non sensibilisés sur la paroi inférieure du container à réaction sur lequel est immobilisée une anti-immunoglobuline, les érythrocytes non sensibilisés étant rassemblés dans le fond du container, l'analyse de l'image finale obtenue étant spécifique de la présence ou non de l'analyte recherché.

Parmi les variantes des techniques utilisées pour le phénotypage ou la RAI, on peut également citer celles qui ont été développées de manière générale pour la recherche dans un échantillon d'un analyte capable de se fixer sur une cellule en utilisant des particules magnétiques, ceci notamment afin de supprimer la centrifugation, opération nécessaire notamment dans les techniques basées sur l'agglutination comme la technique à l'anti-globuline (Coombs indirect par agglutination ou par immunoadhérence sur phase solide) pour la RAI ou encore pour le phénotypage, ou encore, comme pour la RAI, lorsqu'il est nécessaire de laver les globules rouges sensibilisés afin d'éliminer les anticorps non spécifiques capables de reconnaître l'anti-immunoglobuline utilisée à l'étape suivante.

En effet, l'étape de centrifugation est toujours difficile à mettre en oeuvre dans les procédés que l'on souhaite entièrement automatiser, notamment due au coût et à l'encombrement des centrifugeuses, leur manipulation, etc..

Les particules magnétiques sont depuis longtemps utilisées pour la détection de complexes de type ligand-récepteur ou anticorps-antigène, on peut citer par exemple les procédés tels que ceux décrits dans les documents brevets suivants :
- le document WO 92/17781 qui décrit une méthode de détermination de la présence d'un ligand dans un échantillon dans laquelle on incube des particules de latex magnétiques, pouvant être de couleurs différentes, revêtues d'une substance, telle un anticorps, capable de se fixer avec ce ligand, puis on applique un champ magnétique au milieu d'incubation et enfin on observe la présence ou l'absence d'agglutination ; ou encore
- le document EP 0 426 170 qui décrit une méthode de détermination de la présence d'un ligand dans un échantillon, méthode dans laquelle on incube des particules de gélatine magnétiques sensibilisées avec des antigènes ou des anticorps capables de se fixer avec ce ligand, puis on applique un champ magnétique au milieu d'incubation et enfin on observe la présence ou l'absence d'agglutination, ladite méthode étant caractérisée en ce qu'on observe l'état de glissement de ces particules après inclinaison du container, notamment une cupule de microplaque à fond en V.

De telles particules magnétiques ont déjà été utilisées en immunohématologie pour le phénotypage et/ou la RAI. Parmi les documents décrivant de telles applications, on peut citer notamment :
- le document EP 0 351 857 qui décrit un procédé de dosage immunologique utilisant des marqueurs magnétisés tels que des anticorps ou des antigènes fixés sur des billes de latex magnétiques. Ces marqueurs sont capables de se lier à une substance que l'on souhaite déterminer dans une étape d'immunoréaction, et l'on rassemble ensuite les particules magnétiques à marqueur sur une région prédéterminée de la surface d'une paroi dans un récipient de mesure à l'aide d'un aimant positionné sous cette cupule et sous l'action d'un champ magnétique, ce procédé pouvant comprendre une substance qui peut se lier spécifiquement à la substance à déterminer immobilisée sur une région prédéterminée de la surface de la paroi du récipient de mesure. Il est décrit notamment une technique RAI par immunoadhérence dans laquelle des érythrocytes préalablement fixés au fond d'une cupule de microplaque sont ensuite sensibilisés avec le sérum du receveur, puis lavés (par aspiration et injection du liquide de lavage), puis dans laquelle cupule on ajoute les billes de latex magnétiques revêtues d'une anti-immunoglobuline avant d'appliquer un champ magnétique ;
- le document EP 0 528 708 qui décrit un procédé de détection par immunoadhérence d'une substance biologique susceptible d'être présente dans un échantillon. Dans ce procédé, les érythrocytes à phénotyper ou servant de panel de dépistage et/ou d'identification sont fixés préalablement sur le fond d'une microplaque. Après sensibilisation des érythrocytes ainsi fixés avec le sérum test (pour le phénotypage) ou le sérum du receveur à tester (pour la RAI), les cupules sont lavées et on ajoute ensuite des billes de latex magnétiques revêtues d'anti-immunoglobuline. Dans ce procédé, on applique notamment deux types de champ magnétique successifs (de type vertical et circulaire) afin de déplacer les particules magnétiques non liées spécifiquement avec la substance à rechercher ;
- le document brevet EP 0 230 768 qui décrit un procédé de co-agrégation de particules magnétiques capable de se lier à une substance contenue dans un échantillon au moyen de composés polycationiques ou polyanioniques en présence d'un champ magnétique. Ce document décrit en particulier la séparation du plasma d'un échantillon de sang total contenant des globules rouges dans lequel procédé, on ajoute séquentiellement dans un container placé sur un aimant l'échantillon de sang total et un ferrofluide (FeCl₂/FeCl₃) revêtu d'albumine bovine sérique succinylée, les agrégats de particules d'érythrocytes ainsi obtenus étant entraînés vers l'aimant permettant ainsi de récupérer le plasma clarifié par décantation. Dans ce document, il est également décrit un procédé permettant de quantifier la présence d'un anticorps anti-RH (anti-D) dans un échantillon de plasma préparé selon le procédé précédent lequel est incubé en présence d'une suspension de globules rouges RH+ fluorescents et auquel mélange on ajoute ensuite et séquentiellement le ferrofluide succinylé et du polybrène, les globules rouges étant lavés plusieurs fois par application d'un champ magnétique et décantation avant l'ajout d'une anti-immunoglobuline. La quantification de l'anticorps anti-RH dans l'échantillon de plasma est évaluée par comparaison à des témoins en analysant les fluctuations de quantité de fluorescence observée dans un volume donné; et
- Le document brevet US 5 770388 a pour objet un procédé de séparation d'un composé contenu dans un mélange, grâce à l'utilisation de particules magnétiques et de deux liquides de densité différente. Le procédé comprend la mise en contact, dans un premier liquide,du mélange et de particules magnétiques capables de se complexer avec le composé à séparer. Le premier liquide est ensuite déposé sur un second liquide dont la densité est différente de celle du premier, les deux liquides sont soumis à un champ magnétique sous l'effet duquel le complexe composé/ particule magnétique migre vers le second liquide, puis est séparé du second liquide.

Ainsi, il reste de pouvoir disposer d'un procédé rapide et simple de mise en évidence de la présence d'un anticorps dirigé spécifiquement contre un antigène donné dans un mélange réactionnel complexe contenant notamment des anticorps dirigés contre d'autres antigènes, dans lequel procédé, il n'y ait ni étape de lavage et ni étape de centrifugation. Un tel procédé sans étape de centrifugation et sans étape de lavage, notamment pour la RAI, aurait comme avantage de pouvoir être mis en oeuvre sur un support pratique et disponible tel qu'une microplaque, et être entièrement automatisé.

Ceci est justement l'objet de la présente invention.

Dans un réacteur de type cupule de microplaque à fond rond présentant une paroi inclinée revêtue d'une anti-immunoglobuline capable de se lier spécifiquement à un anticorps, les inventeurs ont mis au point un procédé simple et efficace de mise en évidence de complexe spécifique formé entre un anticorps et un antigène dans un mélange réactionnel complexe pouvant contenir des anticorps libres, notamment dirigés contre d'autres antigènes, ceci sans étape de lavage et sans étape de centrifugation. Ce procédé met en oeuvre une suspension de particules magnétiques revêtues d'antigène pouvant être porté par une cellule, ce procédé pouvant être largement automatisé et appliqué en particulier à la RAI, et, le cas échéant, au phénotypage de globules rouges.

Dans ce procédé la présence du complexe anticorps/antigène est déterminée en fin de réaction par visualisation de la présence du complexe formé sur la paroi inclinée du réacteur par immunoadhérence.

Les inventeurs ont mis en évidence de manière surprenante qu'il était possible après incubation d'un mélange réactionnel entre une solution d'anticorps et une suspension de particules magnétiques revêtues d'antigènes, notamment d'érythocytes, ledit mélange réactionnel étant formé dans une cupule au-dessus d'une solution visqueuse ou d'un gel de densité supérieure à la solution d'anticorps, de faire migrer ladite suspension de particules magnétiques au travers de cette solution visqueuse ou de ce gel séparateur vers la paroi inclinée revêtue d'anti-immunoglobuline et/ou le fond de la cupule en utilisant l'action d'un champ magnétique obtenu au moyen d'un aimant situé sous la cupule et de pouvoir visualiser la présence de complexe spécifique formé par immunoadhérence, ceci sans faire migrer les anticorps libres susceptibles de saturer l'anti-immunoglobuline.

Les inventeurs ont mis en évidence de manière encore plus surprenante qu'il était possible d'améliorer très avantageusement ce procédé par une action combinée et simultanée du champ magnétique et d'une agitation rotative de la cupule qui non seulement permet de faciliter grandement la migration des particules, notamment revêtues d'érythrocytes, au travers de la solution visqueuse ou de gel, mais également de favoriser la probabilité de rencontre entre les antigènes portés par les particules magnétiques et l'anti-immunoglobuline fixée sur la paroi inclinée et le fond de la cupule.

Si il a été montré qu'il était possible d'utiliser des solutions visqueuses, ou des gels, de densité supérieure à celle du liquide contenant les anticorps, mais inférieure à celle des érythrocytes, pour séparer par centrifugation des érythrocytes (sensibilisés ou non) des anticorps libres non fixés à ces érythrocytes, ceci pour éviter l'étape de lavage dans les techniques d'immunoadhérence (voir les documents brevets EP 0 058 780 et WO 98/02752 cités précédemment), les inventeurs ont mis en évidence qu'il était également possible d'éliminer également cette étape de centrifugation en utilisant ces substances visqueuses ou gels avec des particules magnétiques porteuses d'antigène, notamment des érythrocytes, au moyen d'un champ magnétique provoqué par un aimant permanent situé à l'extérieur et sous la cupule, en particulier en combinant ce champ magnétique avec une étape d'agitation rotative du réacteur.

Ainsi, sous un premier aspect, la présente invention a pour objet un procédé de mise en évidence d'un complexe spécifique formé par réaction entre un anticorps présent dans une solution et un antigène lié à une particule magnétique, la réaction s'effectuant dans un réacteur ouvert vers le haut et à fond fermé et dont la section diminue au moins dans la zone voisine du fond pour former une paroi inclinée s'étendant jusque dans le fond, ladite paroi inclinée étant au moins en partie revêtue d'une anti-immunoglobuline ou de tout autre composé capable de se lier à l'anticorps dudit complexe formé, caractérisé en ce qu'il comprend les étapes suivantes :
a) préalablement à la réaction :
   - le remplissage préalable du réacteur avec une substance visqueuse ou un gel homogène de manière à recouvrir au moins en partie la paroi inclinée du réacteur, ladite substance visqueuse ou ledit gel ayant une densité telle qu'elle empêche la migration des anticorps n'ayant pas formé de complexe avec les antigènes liés aux particules magnétiques vers la paroi revêtue d'anti-immunoglobuline ou de tout autre composé capable de se lier à l'anticorps lors de l'étape d)
b) la mise en contact au-dessus de la solution visqueuse contenue dans le réacteur de la solution contenant ou susceptible de contenir ledit anticorps avec la suspension de particules magnétiques portant ou susceptible de porter ledit antigène ;
c) l'incubation du réacteur, de préférence pendant un temps et une température appropriés à la formation du complexe, de préférence pendant au moins 5 min ;
d) l'application d'un champ magnétique audit réacteur et l'agitation du réacteur, de telle manière que les particules magnétiques soient entraînées vers le fond et/ou la paroi inclinée du réacteur revêtue d'une anti-immunoglobuline ou de tout autre composé capable de se lier à l'anticorps, en particulier de telle manière que la plus grande partie des particules magnétiques se retrouvent localisées au fond du réacteur et/ou fixées spécifiquement à la paroi inclinée par ladite anti-immunoglobuline ou ledit autre composé capable de se lier à l'anticorps ; et
e) la lecture à l'oeil et/ou par tout autre système de lecture approprié, de l'image obtenue au fond du réacteur et/ou sur la paroi inclinée du réacteur revêtue de ladite anti-immunoglobuline, cette image obtenue permettant de mettre en évidence la présence ou non de la formation d'un complexe spécifique anticorps/antigène.

Par complexe spécifique formé par réaction entre un anticorps présent dans une solution et un antigène lié à une particule magnétique, on entend désigner dans la présente description le complexe formé entre un anticorps capable de reconnaître spécifiquement un antigène et cet antigène, ce complexe étant formé ici sur la particule magnétique sur laquelle est fixé l'antigène.

Par complexe spécifique, il est également inclus ici le complexe formé entre un anticorps capable de reconnaître spécifiquement un antigène porté par une cellule, notamment un érythrocyte et cet antigène, ce complexe étant formé également ici sur la particule magnétique sur laquelle est fixée la cellule porteuse de l'antigène.

De manière générale, l'anticorps du complexe spécifique anticorps/antigène peut être de nature IgG, IgM, IgA ou IgE ou de tout autre classe d'anticorps.

Par anti-immunoglobuline capable de se lier à l'anticorps dudit complexe formé, on entend désigner ici par anti-immunoglobuline, les anti-immunoglobulines, polyclonales ou monoclonales, capables de reconnaître et de fixer tout anticorps, notamment humain, qu'il soit de type IgG, IgM, IgA ou IgE (anti-immunoglobuline totale), ou certaines classes particulières d'anticorps, notamment anti-IgG spécifique. De telles anti-immunoglobulines, notamment humaines, sont bien connues de l'homme de l'art et disponibles chez de nombreux fournisseurs, et ne seront pas développées ici, notamment leurs procédés de production.

Par tout autre composé capable de se lier à l'anticorps dudit complexe formé, on entend particulièrement les composés de type protéine A ou protéine G bien connue de l'homme de l'art pour reconnaître et fixer spécifiquement les anticorps.

Par la plus grande partie des particules magnétiques, on entend désigner ici au moins 60 % de la quantité de particules magnétiques présentes dans le mélange réactionnel, de préférence au moins 70 %, 80 %, 85 %, 90 % et 95 %. De préférence encore au moins 98 %.

De manière particulière lorsque les antigènes sont portés par des cellules, notamment des érythrocytes, par la plus grande partie des particules magnétiques, on entendra désigner la plus grande partie des particules magnétiques ou la plus grande partie des cellules, la plus grande partie des cellules signifiant au moins 60 % de la quantité de cellules magnétisées présentes dans le mélange réactionnel, de préférence au moins 70 %, 80 %, 85 %, 90 % et 95 %. De préférence encore au moins 98 %.

Dans ce cas particulier où les antigènes sont portés par des cellules, notamment des érythrocytes, l'étape d) d'application d'un champ magnétique audit réacteur et d'agitation du réacteur devra être comprise comme de telle manière que les particules magnétiques ou les cellules magnétisées soient entraînées vers le fond et/ou la paroi inclinée du réacteur revêtue d'une anti-immunoglobuline ou de tout autre composé capable de se lier à l'anticorps, en particulier de telle manière que la plus grande partie des particules magnétiques ou des cellules magnétisées se retrouvent localisées au fond du réacteur et/ou fixées spécifiquement à la paroi inclinée par ladite anti-immunoglobuline ou ledit autre composé capable de se lier à l'anticorps.

Dans un mode de réalisation préféré, la présente invention comprend un procédé selon l'invention, caractérisé en ce que le mélange réactionnel obtenu après incubation du mélange entre la solution contenant ou susceptible de contenir ledit anticorps et la suspension de particules magnétiques portant ou susceptible de porter ledit antigène, contient des anticorps libres, en particulier non dirigés contre les antigènes portés par les particules magnétiques, ces anticorps libres étant capables de se fixer à l'anti-immunoglobuline ou au composé utilisé capable de se lier à l'anticorps recherché.

Par anticorps libres, on entend désigner ici les anticorps non complexés à un antigène porté par les particules magnétiques.

Dans un mode de réalisation également préféré, la présente invention comprend un procédé selon l'invention, caractérisé en ce que ledit anticorps contenu ou susceptible d'être contenu dans la solution et dont on cherche à mettre en évidence la formation d'un complexe spécifique avec un antigène porté par les particules magnétiques, est un anticorps de type IgG, ou, si cet anticorps est un anticorps anti-antigène de groupe sanguin, est un anticorps non agglutinant les érythrocytes portant l'antigène correspondant.

De préférence encore, lorsque cet anticorps est de type IgG, on préférera comme anti-immunoglobuline, une anti-IgG, notamment humaine (dirigée contre des anticorps d'origine humaine).

Dans un mode de réalisation particulièrement préféré, la présente invention comprend un procédé selon l'invention, caractérisé en ce qu'à l'étape d), l'agitation du réacteur est effectuée en présence du champ magnétique.

A l'étape d), l'étape d'application du champ magnétique et l'étape d'agitation du réacteur peuvent être réalisées en commençant par l'une ou par l'autre, mais de telle sorte qu'au moins pendant un temps donné, l'application du champ magnétique et l'agitation soient simultanées.

Bien entendu, est également comprise dans le procédé selon l'invention, une variante du procédé selon l'invention dans lequel une application du champ magnétique est effectuée avant l'agitation du réacteur à l'étape d) du procédé, cette application du champ magnétique seule (sans agitation) ne devant pas excéder une durée d'au plus 2 min, de préférence d'au plus 1 min 30 s, 1 min ou 30 s. Il en est de même si l'agitation était effectuée avant l'application du champ magnétique, cette période d'agitation seule ne devant pas excéder une durée d'au plus 2 min, de préférence d'au plus 1 min 30 s, 1 min ou 30 s.

Dans un mode de réalisation particulièrement préféré, la présente invention comprend un procédé selon l'invention, caractérisé en ce qu'à l'étape d), l'application du champ magnétique est effectuée simultanément avec l'agitation du réacteur.

Par simultanément, on entend désigner un laps de temps ne devant pas excéder plus de 20 s où seule une agitation ou une application du champ magnétique est réalisée.

Sous cet aspect, la présente invention comprend un procédé selon l'invention, caractérisé en ce qu'à l'étape d), l'application du champ magnétique et l'agitation sont réalisées simultanément pendant une durée comprise entre 2,5 min et 10 min.

Dans un mode de réalisation préféré, la présente invention comprend un procédé selon l'invention, caractérisé en ce qu'à l'étape d), l'application du champ magnétique et l'agitation sont réalisées simultanément pendant une durée comprise entre 4 min et 7 min, de manière encore plus préférée entre 5 min et 6 min.

La présente invention comprend un procédé selon l'invention, caractérisé en ce qu'à l'étape d), l'application du champ magnétique est réalisée au moyen d'un aimant situé à l'extérieur sous le réacteur de telle manière que les particules magnétiques soient entraînées vers le fond du réacteur, de préférence selon l'axe longitudinal du réacteur.

Dans un mode de réalisation préféré du procédé de l'invention, à l'étape d), ledit aimant est un aimant permanent de magnitude comprise entre 8 000 et 16 000 gauss, de préférence entre 10 000 et 14 000 gauss, de manière plus préférée entre 11 500 et 12 500 gauss, une magnitude de 12 000 gauss étant encore plus préférée.

Dans un mode de réalisation préféré, la présente invention comprend un procédé selon l'invention, caractérisé en ce qu'à l'étape d), l'agitation est réalisée au moyen d'un agitateur rotatif.

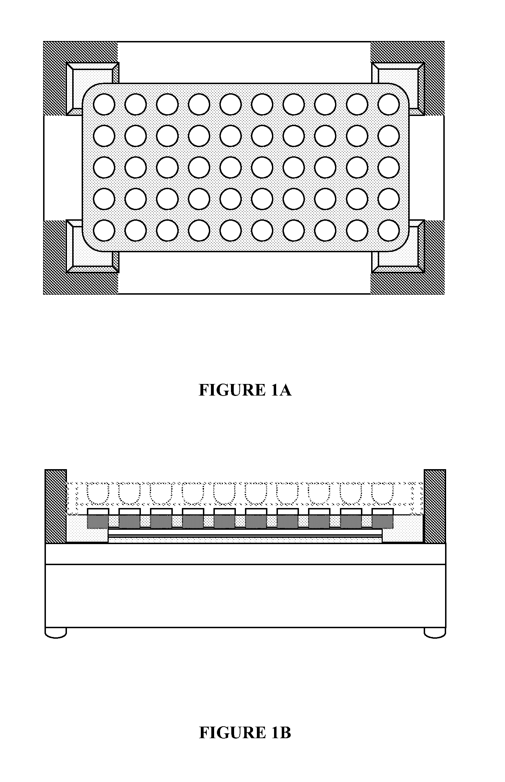

Par agitateur rotatif, on entend citer ici particulièrement une plateforme rotative dont le réacteur, ou l'ensemble des réacteurs lorsqu'il s'agit notamment d'une microplaque de 96 cupules, est solidaire (voir figures 1A et 1B).

Dans un mode particulier d'agitation, la présente invention comprend un procédé selon l'invention, caractérisé en ce qu'à l'étape d), l'agitation est une agitation rotative ayant une orbitale comprise en proportion entre 1,0 mm et 2,5 mm de diamètre, de préférence entre 1,25 mm et 2,25 mm, entre 1,5 mm et 2 mm de diamètre, 2 mm étant le diamètre de l'orbitale préférée, lorsque le réacteur a comme diamètre 7 mm pour sa plus grande section.

Par proportion, on comprendra ici que par exemple lorsque le diamètre de la plus grande section du réacteur est le double ou la moitié de 7 mm, le diamètre correspondant de l'orbitale de rotation mentionné sera respectivement doublé ou divisé par 2.

Par orbitale pour une agitation de type rotative, on entend désigner le diamètre du cercle décrit par le point le plus bas du réacteur lors de l'agitation (point le plus bas de l'axe central longitudinal du réacteur).

Dans un mode de réalisation préféré, la présente invention comprend un procédé selon l'invention, caractérisé en ce qu'à l'étape d), l'agitation est effectuée à une vitesse comprise entre 250 et 750 trs/min, de préférence entre 400 et 600 trs/min.

Dans un mode de réalisation particulier, l'aimant situé sous chacun des réacteurs est solidaire de la plateforme d'agitation (voir également figures 1A et 1B).

Dans ce dernier cas, l'axe central longitudinal de l'aimant situé sous le réacteur suit l'orbitale suivie par l'axe central longitudinal du réacteur pendant l'agitation.

Dans un mode de réalisation particulier, l'aimant situé sous chacun des réacteurs est désolidarisé de la plateforme d'agitation. Dans ce dernier cas, l'axe central longitudinal de l'aimant situé sous le réacteur ne bouge pas et ne suit pas l'orbitale suivie par l'axe central longitudinal du réacteur pendant l'agitation.

La présente invention comprend un procédé selon l'invention, caractérisé en ce qu'à l'étape c), la durée d'incubation est comprise entre 10 min et 30 min, de préférence entre 15 min et 25 min.

Dans un mode de réalisation préféré, la présente invention comprend un procédé selon l'invention, caractérisé en ce qu'à l'étape c), l'incubation est réalisée à une température comprise entre 10°C et 40°C, de préférence entre 25°C et 40°C, entre 30°C et 40°C, de préférence à environ 37°C (37°C ± 1°C).

Dans un mode de réalisation préféré, la présente invention comprend un procédé selon l'invention, caractérisé en ce qu'à l'étape b), l'incubation est réalisée à une température comprise entre 30°C et 40°C, de préférence à 37°C.

Les techniques immunodiagnostiques, de capture ou de tri cellulaire mettant en oeuvre des particules magnétiques ont fait l'objet de nombreuses publications et sont bien connues de l'homme de l'art.

Parmi ces techniques, on peut citer celles faisant appel à une fonctionnalisation de la particule magnétique permettant d'obtenir une fonction réactive en surface capable de réagir dans des conditions et avec des réactifs appropriés avec l'antigène que l'on souhaite greffer de manière covalente sur la particule, notamment une fonction acide, amine, époxy ou aldhéhyde pour ne citer que les plus courants.

On peut également citer celles faisant appel à une adsorption passive de l'antigène que l'on souhaite fixer sur la particule, notamment par un traitement adéquate permettant d'obtenir des billes chargées positivement ou négativement en fonction des antigènes et des conditions dans lesquelles on souhaite réaliser cette absorption passive.

Parmi les fournisseurs, dont les microbilles (ou particules) magnétiques peuvent être utilisées dans le cadre de la présente invention, on peut citer en particulier la société Ademtech (33600 Pessac, France) qui propose des particules magnétiques d'environ 100 à 500 nm de diamètre pouvant être fonctionnalisées par une acide ou une amine ainsi que les protocoles et réactifs permettant d'effectuer le greffage souhaité avec ces fonctions. Cette société fournit également ces mêmes particules non fonctionnalisées, de type hydrophobe de diamètre d'environ 300 nm ± 30 nm, particules utilisées notamment dans les exemples ci-après. Ces particules magnétiques sont constituées à plus de 50 % d'un noyau de composé ferromagnétique (type oxyde de fer), ce noyau étant enrobé de polystyrène. On peut également citer la société Bioclone Inc. (San Diego, CA, U.S.A.) qui propose tout un catalogue de billes magnétiques fonctionnalisées pouvant être utilisées. On peut citer encore la société Dynal Biotech GmbH (Hamburg, Germany) avec sa gamme très variée de Dynabeads™ proposant notamment des microbilles magnétiques activées à la streptavidine, au tosyl ou activées par une fonction acide carboxylique. On peut également citer la société Merck Chimie SAS (94126 Fontenay-sous-Bois, France) qui avec sa gamme de microbilles magnétiques Estapor™ propose différentes tailles de particules (de 300 nm à 2 µm) à base de polystyrène ou de divinylbenzène pouvant comprendre jusqu'à plus de 50 % de ferrite et pouvant être fonctionnalisées ou non, par exemple avec une fonction acide ou amine. Ces particules sont préparées par un procédé mettant en oeuvre une polymérisation du styrène en présence du composé ferromagnétique. On peut enfin citer les particules magnétiques décrites dans le document brevet européen EP 0 038 730 de Rhône Poulenc qui décrit des latex de polymères magnétiques, dans le document brevet européen EP 0 125 995, dans les demandes de brevets européens EP 0 105 714, EP 0 190 006, EP 0 238 353 et EP 0 249 357 de Serono Diagnostics, ou encore dans les documents brevets français FR 2 262 805 et FR 2 454 098 de Corning Glass Works.

Par particules magnétiques au sens de la présente invention, on entend également ici les solutions aqueuses de ferrofluide obtenues à partir d'un mélange polyoxoanions de Fe(III) et d'au moins un métal M(II) de degré II d'oxydation, tel que Fe(II)), ferrofluide tel qu'obtenu notamment par les procédés décrits aux exemples 1 à 8 de la demande de brevet français publiée sous le No. FR 2 461 521.

De préférence la solution de ferrofluide qui pourra être utilisée dans les procédés de l'invention résultera d'une dilution de la solution de ferrofluide, obtenu par les procédés de préparation de ferrofluide décrits aux exemples 1 à 8 de la demande de brevet français publiée sous le No. 2 461 521, en milieu aqueux et de préférence en absence de tensio-actif (ou détergent) pour éviter notamment la lyse des érythrocytes lorsque ces derniers seront mis en contact avec cette solution de ferrofluide pour être magnétisés, ceci dans le procédé particulièrement préféré de l'invention mettant en oeuvre des antigènes portés par des érythrocytes.

Dans un mode de réalisation préféré, ladite solution de ferrofluide sera caractérisée en ce qu'elle est préparée à partir d'un mélange polyoxoanions de Fe(III) et d'au moins un métal M(II) de degré II d'oxydation de préférence choisi parmi les métaux de la première série des métaux de transition tels que Fe(II), Co(II), Mn(II), Cu(II) ou Ni(II).

La solution de ferrofluide pourra notamment être préparée à partir d'un mélange de polyoxoanions de Fe(III) et d'au moins un métal M(II) associé à un cation tel que H⁺, CH₃⁺, N(CH₃)₄⁺, N(C₂H₅)₄⁺ ou tout autre cation capable de conférer au polyoxoanion une plus grande solubilité dans l'eau que les cations Na, K⁺ et NH₄⁺, ces cations pourront être notamment apportés par les acides appropriés, tels que HCl, CH₃COOH, ou encore par l'hydroxyde de tétraméthyl- ou tétraéthyl-ammonium.

De préférence, les sources de métaux de départ choisis pour Fe(III) et M(II) pour préparer le ferrofluide seront des sels choisis parmi :
- pour Fe(III), ceux listés dans la demande de brevet français publiée sous le No. 2 461 521, page 4, lignes 1 et 2, notamment le chlorure ferrique, et
- pour M(II), ceux listés dans la demande de brevet français publiée sous le No. 2 461 521, page 4, lignes 3 à 6, notamment le chlorure ferreux.

De préférence, la solution de ferrofluide est préparée à partir d'un mélange entre Fe(III) et le métal M(II) caractérisé en ce que le rapport molaire initial de degré II est de 2 ± 1 ; de manière plus préférée de 2 ± 0, 5 ; 2 ± 0,25 ou encore 2 ± 0,1, un rapport molaire initial de 2 entre Fe(III) et le métal M(II) de degré II étant le plus préféré.

De préférence encore, au mélange initial de sel de Fe(III) et du métal M(II) de degré II sera ajoutée une base forte appropriée telle que l'hydroxyde de sodium, l'hydroxyde de tétraméthyl- ou tétraéthyl-ammonium.

De préférence encore, la solution aqueuse de ferrofluide est préalablement diluée dans un tampon ou une solution physiologique.

Dans certaines applications, notamment lorsqu'il s'agira d'améliorer la sensibilité ou la vitesse de réaction (sans toutefois augmenter la non spécificité du test), on pourra utiliser un tampon dit de basse force ionique (BFI), appelée encore tampon LISS (« LISS » pour « Low Ionic Strength Solution »).

Dans un mode de réalisation préféré, ladite solution de ferrofluide est diluée de 0,25 à 10 % (v/v) dans ledit tampon ou ladite solution physiologique, de préférence entre 0,25 et 5 %, entre 0, 25 et 2,5 %, entre 0,25 et 1 %, entre 0,25 et 0,75 %.

Dans un mode de réalisation préféré, la présente invention comprend un procédé selon l'invention, caractérisé en ce que les particules magnétiques ont un diamètre compris entre 100 nm et 1,5 µm, de préférence entre 150 nm et 1,2 µm, un diamètre compris entre 200 nm et 1 µm ou entre 200 nm et 800 nm étant le plus préféré.

Dans un mode de réalisation préféré, la présente invention comprend un procédé selon l'invention, caractérisé en ce que les particules magnétiques contiennent au moins 35 %, de préférence au moins 50 % en poids de composés ferromagnétiques (ferrite ou oxyde de fer par exemple), de préférence au moins 70 %, de préférence lesdits composés ferromagnétiques sont des oxydes de fer.

De préférence encore, lorsque l'antigène du complexe spécifique est porté par une cellule, notamment un érythrocyte, les particules magnétiques sont de type hydrophobe et lavées préalablement en présence de surfactant, de préférence d'un détergent de type non-ionique.

La présente invention comprend un procédé selon l'invention, caractérisé en ce qu'à l'étape a), le remplissage préalable du réacteur avec une substance visqueuse ou un gel homogène est réalisé avec une substance visqueuse ou un gel dont la densité est telle qu'elle empêche la migration des anticorps n'ayant pas formé de complexe avec les antigènes liés aux particules magnétiques vers la paroi inclinée et le fond du réacteur revêtu d'anti-immunoglobuline ou du composé capable de reconnaître l'anticorps lors de l'étape d).

Dans un mode de réalisation préféré, la présente invention comprend un procédé selon l'invention, caractérisé en ce qu'à l'étape a), la solution visqueuse ou le gel est de densité supérieure à 1. De préférence, la solution visqueuse est une solution à base de sérum albumine, en particulier bovine, à base de polyvinyl pyrrolidone (PVP-40 ou PVP-60) ou encore de gélatine.

L'homme de l'art saura adapter le protocole exemplifié ci-après dans les exemples pour une solution homogène de gel Sephadex™ G-100 superfine aux autres types de gel ou de solution visqueuse, ceci suivant la nature des antigènes (portés ou non par des cellules), la taille et la composition des particules magnétiques en ferrite, ceci pour obtenir la migration complète des particules au travers de ces solutions visqueuses ou gels, notamment en faisant varier la densité finale de la solution visqueuse ou de gel, le temps de migration (agitation), la vitesse d'agitation et le cas échéant l'orbitale de rotation de l'agitation rotative.

Par exemple, en ce qui concerne les solutions visqueuses à base d'albumine ou de PVP, des concentrations d'environ 30 % ± 10 % pourront être utilisées.

Dans un mode de réalisation préféré, la présente invention comprend un procédé selon l'invention, caractérisé en ce qu'à l'étape a), le gel est un gel de dextran ou d'agarose (Type Sepharose™ (Pharmacia, Suède), notamment le Sepharose™ 4B ou 6B).

Dans un mode de réalisation également préféré, lorsque ladite solution est de type gel, ce gel est préparé en présence de sérum albumine bovine afin d'augmenter la densité de la solution de gel, de préférence à des concentrations finales dans la solution de gel comprises entre 5 % et 15 % en p/v, de préférence à 10 % ± 2,5 %.

Dans un mode de réalisation préféré, la présente invention comprend un procédé selon l'invention, caractérisé en ce qu'à l'étape a), la solution visqueuse ou gel est du Sephadex™, (Société Pharmacia, Suède, ou Sigma-Aldrich), de préférence du G-10™, du G-25™, du G-50™, du G-75™, du G-100™, du G-150™ ou du G-200™, dont le diamètre des billes de dextran peut varier entre 20 nm et 300 nm. De manière plus préférée le Sephadex™ est le G-100™ superfine.

De préférence encore, la concentration en gel, notamment en Sephadex™ ou Sepharose™ est comprise entre 1,5 % et 6 %, de préférence entre 2 % et 5 %, entre 2,5 % et 4 %. La concentration de 3 % ± 0,5 % en p/v étant la plus préférée, notamment pour le Sephadex™, en particulier pour le G-100™ superfine.

Dans un mode de réalisation préféré, la présente invention comprend un procédé selon l'invention, caractérisé en ce qu'à l'étape b), la solution contenant ou susceptible de contenir ledit anticorps est déposée sur la solution visqueuse avant le dépôt de la suspension de particules magnétiques portant ou susceptible de porter ledit antigène.

Dans un mode de réalisation préféré, la présente invention comprend un procédé selon l'invention, caractérisé en ce qu'avant l'étape b), la solution visqueuse ou de gel est recouverte d'une solution aqueuse destinée à diluer le mélange réactionnel déposé à l'étape b), de préférence lorsque la solution d'anticorps est du sérum ou plasma humain non dilué. De préférence, ladite solution aqueuse destinée à diluer la solution ou l'échantillon d'anticorps représente un volume de 1 à 10 fois le volume de la solution d'anticorps, de préférence encore de 2 à 7 fois, de 3 à 6 fois ou de 4 à 5,5 fois. De 4 à 5 fois étant la gamme la plus préférée, notamment dans un réacteur de type cupule de microplaque 96 puits. La solution de dilution peut être ainsi une solution physiologique, pouvant également contenir de la BSA (voir ci-après pour les concentrations de BSA préférées).

Dans un mode de réalisation préféré, la présente invention comprend un procédé selon l'invention, caractérisé en ce qu'avant l'étape b), la solution visqueuse ou de gel est recouverte d'une solution aqueuse destinée également à empêcher le contact direct de l'échantillon contenant les anticorps à tester (plasma ou sérum) avec la paroi de la cupule recouverte d'AGH ou à empêcher le mélange de cet échantillon avec la solution visqueuse ou de gel, ceci pour éviter toute réaction ou saturation des AGH coatée sur le fond de la paroi avec des anticorps non spécifiques contenus dans ledit échantillon de plasma ou de sérum, réaction ou saturation qui empêcherait la fixation ultérieure du complexe spécifique anticorps/antigène que l'on cherche à détecter.

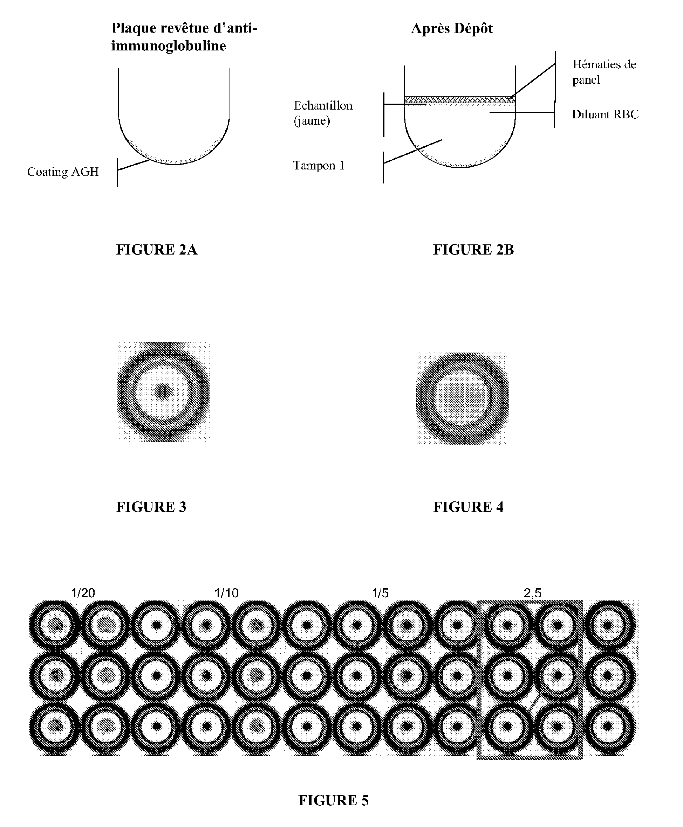

Dans un mode de réalisation préféré, la présente invention comprend un procédé selon l'invention, caractérisé en ce qu'avant l'étape b), la solution visqueuse ou de gel est recouverte d'une solution aqueuse destinée à diluer et à favoriser la formation du complexe anticorps/antigène à l'étape b) (voir figures 2A et 2B).

Dans ce cas, lorsqu'il s'agit de mettre en évidence un complexe spécifique formé par un anticorps/antigène de groupe sanguin, notamment dans le cadre d'une RAI ou d'un phénotypage de globule rouge, cette solution de dilution favorisant la réaction est réalisée à base d'un tampon basse force ionique (BFI), ce tampon étant bien connu de l'homme de l'art en immunohématologie, ce tampon BFI pouvant être le cas échéant réalisé en présence d'une concentration finale en sérum albumine bovine (BSA) comprise entre 1,5 % et 6 % (p/v), de préférence entre 2 % et 5 %, la valeur 3 % ± 1 % étant préférée.

Dans certaines applications, notamment lorsqu'il s'agira d'améliorer la sensibilité ou la vitesse de réaction (sans toutefois augmenter la non spécificité du test), on pourra utiliser un tampon dit de basse force ionique (BFI), appelé encore tampon LISS (« LISS » pour « Low Ionic Strength Solution »).

Par tampon ou solution physiologique, l'homme de l'art comprendra qu'il s'agit ci-avant de tampon habituellement utilisé dans le domaine de la biologie cellulaire, notamment en immunohématologie afin d'éviter la lyse des globules rouges, notamment pour l'application RAI ou phénotypage. De tels tampons ou solutions seront par exemple des tampons à pH physiologique, compris entre 6,8 et 7,5, avec une molarité des constituants du tampon ajustée de telle sorte que la solution finale obtenue s'apparente pour la pression osmotique à la molarité d'une solution de type NaCl à 9 pour mille (proche de 0,15 M de NaCl). On peut notamment citer mais sans s'y limiter le tampon phosphate type PBS à pH 7,0 - 7,4 bien connu de l'homme de l'art.

La composition des tampons BFI ou LISS ne sera pas ici développée, ces tampons étant bien connus en immunohématologie pour favoriser les réactions d'agglutination. Ces tampons sont notamment disponibles auprès des fournisseurs de réactifs pour l'immunohématologie (on pourra citer pour exemple, mais sans s'y limiter le tampon LISS de composition suivante : 16 g/l de glycine, 0,03 M de NaCl et 0,015 M phosphate à pH 6,7).

Dans un mode de réalisation préféré, la présente invention comprend un procédé selon l'invention, caractérisé en ce que le réacteur est une cupule de microplaque à fond rond (ou encore appelé à fond en U ou hémisphérique), ou encore ou à fond V.

Les réacteurs de type cupule de microplaque présensibilisée (revêtue) sur le fond et sur en partie la paroi inclinée de la cupule avec l'anti-immunoglobuline humaine ou tout autre composé capable de reconnaître un anticorps peuvent être réalisés aisément selon les techniques bien connues de l'homme de l'art (voir notamment les exemples ci-après).

Dans un mode de réalisation préféré, la présente invention comprend un procédé selon l'invention, caractérisé en ce que la solution d'anticorps est un échantillon de plasma ou de sérum humain dans lequel on cherche à mettre en évidence la présence d'un anticorps dirigé spécifiquement contre un antigène lié à la particule magnétique, de préférence un anticorps n'agglutinant pas les particules magnétiques, en particulier de type IgG, et en ce que l'anti-immunoglobuline est une anti-immunoglobuline humaine, notamment une anti-IgG humaine.

Dans un mode de réalisation préféré, la présente invention comprend un procédé selon l'invention, caractérisé en ce qu'à l'étape e), la collecte au point le plus bas du réacteur de l'ensemble des particules magnétiques est significative de l'absence de formation de complexe spécifique anticorps/antigène ou en ce que la présence d'au moins une fraction visible de particules magnétiques sur la paroi inclinée du réacteur revêtue d'anti-immunoglobuline est significative de la formation dudit complexe.

De préférence, les particules magnétiques peuvent être colorées pour une meilleure visualisation de ces particules.

Dans un mode de réalisation préféré, la présente invention comprend un procédé selon l'invention, pour la mise en évidence d'un complexe spécifique formé par réaction entre un anticorps présent dans une solution et un antigène porté par une cellule ou par un virus, elle- ou lui-même lié à une ou plusieurs particules magnétiques (incluant ici les solutions de type ferrofluide comme ci-avant mentionnées) suivant la taille de la cellule ou du virus et celle de la particule.

De préférence, la cellule est choisie parmi les cellules eucaryotes, mammifère ou levure, ou de bactérie.

De préférence encore, la cellule est une cellule de mammifère, notamment humaine, de préférence un globule blanc, de type lymphocyte ou macrophage, ou encore une plaquette ou encore un érythrocyte (ou globule rouge).

Sous un aspect particulier l'antigène porté par la cellule n'est pas un antigène naturellement présent à la surface externe de la cellule, cet antigène ayant dans ce cas était préalablement fixé par liaison covalente, ionique ou adsorbé à la surface de la cellule.

Dans un mode de réalisation préféré, la présente invention comprend un procédé selon l'invention, pour la mise en évidence d'un complexe spécifique formé par réaction entre un anticorps anti-antigène de groupe sanguin présent dans une solution et un antigène de groupe sanguin, caractérisé en ce que la cellule est un érythrocyte, et de préférence en ce que ledit antigène est un antigène naturel porté par l'érythrocyte.

Dans la présente description les termes érythrocyte, globule rouge et hématie seront employés indifféremment pour désigner la même cellule sanguine.

Dans un mode de réalisation préféré, la présente invention comprend un procédé selon l'invention, pour la recherche d'agglutinines irrégulières (RAI) ou la recherche d'anticorps érythrocytaires dans un échantillon de sérum ou plasma, de préférence d'anticorps non agglutinants, ou encore pour le phénotypage de globule rouge, caractérisé en ce qu'il comprend une étape dans laquelle les érythrocytes portant les antigènes sont préalablement liés par liaisons ioniques ou par adsorption à des particules magnétiques ou en suspension dans une solution de ferrofluide, de préférence les particules magnétiques sont non fonctionnalisées et lavées en présence de surfactant, notamment non ioniques, ces particules magnétiques pouvant être après ce lavage en détergent mises en contact avec une solution d'albumine (comprise entre 0,05 % et 5 %, de préférence entre 0,1 % et 1 %) pour faciliter l'adsorption des érythrocytes.

Dans ce cas, l'albumine est elle-même liée par liaison ionique ou adsorbée à la surface de la particule magnétique si le traitement de cette particule ou sa fonctionnalisation, notamment sa charge surfacique résultante, autorise cette liaison ionique ou cette adsorption ; ou encore par couplage covalent de l'albumine sur des fonctions de la particule magnétique, notamment carboxyliques ou aldéhydiques greffées à la surface de ces particules autorisant notamment la possibilité de formation de liaison covalente avec les fonctions amines, notamment avec des résidus lysines de l'albumine (BSA).

Dans un mode de réalisation préféré, la présente invention comprend un procédé selon l'invention, pour le phénotypage de globules rouges, caractérisé en ce que la solution d'anticorps à l'étape b) est une solution de sérum test contenant un anticorps anti-antigène de groupe sanguin connu, de préférence de nature IgG, le réacteur étant alors revêtu d'une anti-immunoglobuline dirigée contre l'espèce dont est issu l'anticorps anti-antigène de groupe sanguin utilisé (animale ou humaine) ou de tout autre composé capable de reconnaître l'anticorps spécifique contenu dans ce sérun test (notamment une protéine A ou G).

Dans un mode de réalisation préféré, la présente invention comprend un procédé selon l'invention, caractérisé en ce qu'avant ou après leur fixation sur des particules magnétiques ou leur mise en suspension dans la solution de ferrofluide, de préférence avant, les érythrocytes peuvent être soumis à l'action d'une protéase, de préférence la broméline ou la papaïne, ceci afin de favoriser la formation du complexe spécifique, ces techniques étant bien connues de l'homme de l'art pour le phénotypage de groupe sanguin.

Dans un mode de réalisation préféré, la présente invention comprend un procédé selon l'invention, caractérisé en ce que la concentration d'érythrocytes dans la suspension finale d'érythrocytes liés aux particules magnétiques ou dans la solution de ferrofluide, avant d'être ajoutée à la solution d'anticorps dans le réacteur, est comprise entre 0,1 % et 5 % en volume, de préférence entre 0,5 % et 2,5 % et entre 0,75 % et 2 %, la valeur 1 % ± 0, 25 % étant la plus préférée.

Dans un procédé de l'invention particulièrement préféré pour la RAI et/ou le phénotypage réalisé dans une cupule de microplaque, ce procédé comprend les étapes suivantes :
a) préalablement à la réaction :
   - le remplissage préalable d'une cupule fond rond avec 50 µl ± 10 µl d'une solution homogène d'un gel de Sephadex™ G-100 superfine à environ 3 % ± 0,5 % dans un tampon de type LISS en présence de BSA à 10 % ± 2,5 % de manière à recouvrir au moins en partie la paroi inclinée du réacteur ; et, le cas échéant,
   - l'ajout de 60 µl ± 15 µl d'un tampon LISS à 3 % ± 1 % de BSA, de préférence à pH compris entre 6,5 et 7,5 ;
   - de disposer d'érythrocytes magnétisés à l'aide d'une solution de ferrofluide ou particules magnétiques, de préférence quand il s'agit de particules magnétiques de taille comprise entre 200 nm et 1000 nm ou entre 200 nm et 800 nm, de préférence ces particules sont non fonctionnalisées, de préférence contiennent au moins 40 % de ferrite (d'oxyde de fer), de préférence encore étant recouvertes de BSA et/ou ayant été préalablement traitées afin que ces particules puissent se fixer par liaison ionique ou par adsorption sur les érythrocytes portant l'antigène pouvant former le complexe spécifique avec ledit anticorps, notamment par lavage en présence de détergent non ionique ;
b) la mise en contact au-dessus de la solution de gel homogène, ou, le cas échéant, au-dessus du tampon LISS à 3 % ± 1 % de BSA, contenue dans la cupule :
   - de la solution contenant ou susceptible de contenir ledit anticorps avec 25 µl ± 7,5 µl de la suspension d'érythrocytes revêtus de particules magnétiques portant ou susceptibles de porter ledit antigène, les érythrocytes étant à une concentration dans la suspension comprise entre 0,75 % et 1,25 % en v/v, avec
   - 12 µl ± 8 µl d'échantillon contenant les anticorps (sérum ou plasma dans le cas de la RAI, ou sérum test dans le cas d'un phénotypage) ;
c) l'incubation du réacteur pendant 20 min ± 5 min à 37°C ± 1°C ;
d) simultanément, l'application d'un champ magnétique audit réacteur et l'agitation du réacteur, pendant une durée de 5 min 30 s ± 1 min ; et
e) la lecture à l'oeil et/ou par tout autre système de lecture approprié, de l'image obtenue au fond du réacteur et/ou sur la paroi inclinée du réacteur revêtue de ladite anti-immunoglobuline, cette image obtenue permettant de mettre en évidence la présence ou non de la formation d'un complexe spécifique anticorps/anti-antigène, l'interprétation pouvant être réalisée à l'aide de la figure 3 et de la figure 4 caractéristique respectivement d'une image négative (pas de formation de complexe spécifique et d'une image positive (présence de complexe).

Sous un autre aspect, la présente invention a pour objet un dispositif pour la mise en évidence d'un complexe spécifique formé par réaction entre un anticorps présent dans une solution et un antigène lié à une particule magnétique, de préférence pour la mise en évidence d'un complexe spécifique formé par réaction entre un anticorps anti-antigène de groupe sanguin présent dans une solution et un antigène de groupe sanguin porté par un érythrocyte lié lui-même à une ou plusieurs particules magnétiques, notamment pour la RAI, la recherche d'anticorps érythrocytaire, ou encore pour le phénotypage de groupe sanguin, caractérisé en ce qu'il comprend :
a)
   - un réacteur ou un ensemble de réacteurs ouvert vers le haut et à fond fermé et dont la section diminue au moins dans la zone voisine du fond pour former une paroi inclinée s'étendant jusque dans le fond, ladite paroi inclinée étant au moins en partie revêtue d'une anti-immunoglobuline ou d'un composé capable de se lier à l'anticorps dudit complexe formé,
   - chacun des réacteurs pouvant être rempli en partie d'une substance visqueuse ou d'un gel, ladite substance visqueuse ou ledit gel ayant une densité telle qu'elle empêche la migration des anticorps n'ayant pas formé de complexe avec les antigènes liés aux particules magnétiques vers la paroi revêtue d'anti-immunoglobuline ou de tout autre composé capable de se lier à l'anticorps ;
b) au moins un aimant ou un ensemble d'aimants pouvant être disposé à l'extérieur sous le ou les réacteurs et un système d'agitation rotatif dudit ou desdits réacteurs, ce système d'agitation étant de préférence solidarisé avec l'aimant ou la plate-forme supportant les aimants afin de déplacer les aimants de la même manière que lesdits réacteurs pendant la phase d'agitation ;
c) le cas échéant, un incubateur capable de réguler la température d'incubation des réacteurs ; et
d) le cas échéant, un lecteur capable d'évaluer la présence et la localisation des érythrocytes magnétisés à la fin de la réaction sur chacun des réacteurs, notamment sur la paroi inclinée et le fond du réacteur revêtu de l'anti-immunoglobuline ou du composé capable de se lier à l'anticorps du complexe spécifique éventuellement formé.

De préférence, le dispositif selon l'invention est caractérisé en ce que la substance visqueuse ou le gel a les caractéristiques telles que définies dans le procédé selon l'invention ci-avant avec les mêmes préférences. Il en est de même pour les particules magnétiques ou la suspension de cellules magnétisées, les caractéristiques de l'aimant et de l'agitateur rotatif.

De préférence encore, le dispositif selon l'invention est caractérisé en ce que ledit réacteur est une cupule de microplaque, de préférence à fond rond (hémisphérique) ou à fond en V, une microplaque comprenant 96 cupules étant encore plus préférée.

De préférence, l'aimant est sous la forme d'une pile, comme montré au schéma des figures 1A et 1B, de préférence une plate-forme comprenant 96 aimants en forme de pile, disposés chacun sous une cupule.

Sous encore un autre aspect, l'invention concerne un kit pour la mise en évidence d'un complexe spécifique formé par réaction entre un anticorps présent dans une solution et un antigène lié à une particule magnétique, caractérisé en ce qu'il comprend :
a) un réactif comprenant une suspension de particules magnétiques revêtues au moins d'un antigène ou destinées à être revêtues d'au moins un antigène ; et
b)
   - un réacteur ou un ensemble de réacteurs ouvert vers le haut et à fond fermé et dont la section diminue au moins dans la zone voisine du fond pour former une paroi inclinée s'étendant jusque dans le fond, ladite paroi inclinée étant au moins en partie revêtue d'une anti-immunoglobuline capable de se lier à l'anticorps dudit complexe formé,
   - un container contenant une solution visqueuse ou un gel, ou le cas échéant, chacun des réacteurs étant rempli en partie de ladite substance visqueuse ou dudit gel, ladite substance visqueuse ou ledit gel ayant une densité telle qu'elle empêche la migration des anticorps n'ayant pas formé de complexe avec les antigènes liés aux particules magnétiques vers la paroi revêtue d'anti-immunoglobuline ou de tout autre composé capable de se lier à l'anticorps ; et,
c) le cas échéant, au moins un aimant ou un ensemble d'aimants pouvant être disposé à l'extérieur sous le ou les réacteurs, ledit aimant étant de préférence solidaire d'un agitateur rotatif, de préférence l'orbitale de l'agitateur étant de 2 mm ± 1 mm pour un diamètre de réacteur dans sa plus grande section de 7 mm (ou en proportion équivalente).

De préférence, l'invention concerne un kit selon l'invention, pour la RAI, caractérisé en ce que ledit réactif contient une suspension d'érythrocytes tests de phénotype connu sur lesquels érythrocytes sont adsorbées ou couplées lesdites particules magnétiques, ou caractérisé en ce qu'il comprend un second réactif contenant une suspension d'érythrocytes tests de phénotype connu destinés à être adsorbés ou couplés sur les particules magnétiques ou ferrofluide contenues dans le premier réactif.

De préférence, l'invention concerne un kit selon l'invention, pour le phénotypage de groupe sanguin caractérisé en ce que ledit réactif contient un sérum test contenant un anticorps anti-antigène de groupe sanguin, de préférence de type IgG, et, en outre un réactif contenant une suspension de particules magnétiques ou solution de ferrofluide pouvant se lier à une suspension d'érythrocytes que l'on cherche à phénotyper.

De préférence également, l'invention concerne un kit selon l'invention, caractérisé en ce que la substance visqueuse ou le gel, et le cas échéant les particules magnétiques ou la suspension de cellules magnétisées, le cas échéant les caractéristiques de l'aimant et de l'agitateur rotatif, a ou ont les caractéristiques telles que définies pour le procédé selon l'invention avec également les préférences définies pour ces solutions visqueuses ou de gel ou autres composés et éléments.

De préférence encore, l'invention concerne un kit selon l'invention, caractérisé en ce que ledit réacteur est une cupule de microplaque, de préférence à fond rond (hémisphérique) ou à fond en V, une microplaque comprenant 96 cupules étant encore plus préférée.

Les figures dont les légendes sont ci-après, ainsi que les exemples qui suivent sont destinés à illustrer l'invention sans aucunement en limiter la portée.

### Légendes des figures

**Figures 1A et 1B** : Schéma vue de dessus (figure 1A) et vue de côté (figure 1B) de la plate-forme d'agitation (« teleshake™ ») comprenant une plaque de fer doux sous la microplaque sur laquelle plaque de fer doux est fixé magnétiquement chacun des aimants en forme de pile sous chaque cupule de la microplaque.
   (1) Gabarit en plastique permettant d'obtenir le bon espacement des aimants ; (2) Eléments permettant de caler la plaque d'aimants sur le teleshake ; (3) Taquet du Teleshake permettant de caler les microplaques ; (4) Aimant piles ; (5) Microplaque ; (6) Gabarit plastique ; (7) Plaque de fer doux sur laquelle les aimants sont fixés magnétiquement ; (8) Plaque de carton isolant le teleshake du champ magnétique des aimants piles ; (9) Plateau rotatif du teleshake.
**Figures 2A et 2B** : Schémas d'une cupule réacteur à fond rond (ou en U) après revêtement avec de l'anti-immunoglobuline sur sa paroi inclinée (en partie) et au fond de la cupule avant l'ajout de la solution de gel (tampon 1) et des différents solutions et réactifs.
**Figure 3** : Photographie montrant une image d'une réaction négative d'immunoadhérence avec des antigènes portés par des érythrocytes (pas de formation de complexe spécifique).
**Figure 4** : Photographie montrant une image d'une réaction positive d'immunoadhérence avec des antigènes portés par des érythrocytes (formation de complexe spécifique adhérent à la paroi inclinée revêtue d'anti-immunoglobuline).
**Figure 5** : Titrage de l'anti-D du CNRGS, dilution en polycontrol : douteux à 1 croix sur l'hématie homozygote à 2,5 ng/ml.
**Figure 6** **:** Titrage de l'anti-D du CNRGS (Centre National de Référence des Groupes Sanguins), dilution en plasma AB : 1 croix sur l'hématie homozygote à 2,5 ng/ml.
**Figure 7** **:** Titrage de l'anti- D et de l'anti- Fya du Contrôle Qualité en sérum AB. *
**Figure 8** **:** Répétabilité et Reproductibilité
   8 échantillons négatifs et 4 échantillons positifs (anti-D) ont été passés 3 fois sur une même plaque.
**Figure 9** **:** Répétabilité et Reproductibilité
   8 échantillons négatifs et 4 échantillons positifs (anti-D) ont été passés sur une autre plaque.
**Figure 10** **:** Photographie au microscope électronique d'une hématie Hemascreen™ magnétisée à l'aide de particules magnétiques de diamètres moyen compris entre 500 et 750 nm, prêtes à l'emploi.
**Figure 11** **:** Résultats obtenus en cupule de microplaque pour l'évaluation de la sensibilité de la méthode RAI Qwalys™ sur un échantillon test anti-RH1 de référence internationale.
**Figure 12** **:** Résultats typiques obtenus en cupule de microplaque pour la RAI sur des échantillons de plasma ou de sérum de patients avec un panel de 3 hématies selon la technologie Qwalys ™ de l'invention.
**Figure 13** **:** Résultats obtenus en cupule de microplaque pour l'évaluation de la sensibilité de la méthode RAI Qwalys ™ sur un échantillon test anti-RH1 de référence internationale.

### EXEMPLE 1 : Revêtement de la paroi inclinée et du fond de la cupule avec une anti- immunoglobuline

La nature chimique et physico-chimique du plastique de la cupule utilisée permet de recouvrir celui-ci d'une couche d'anti-immunoglobuline humaine (de type AHG monoclonale ou polyclonale) capable de se lier spécifiquement avec les anticorps des complexes spécifiques éventuellement formés, lorsque l'anticorps dudit complexe est d'origine humaine.

On peut noter en outre que cette composition d'AGH peut comprendre des anticorps dirigés contre des déterminants de protéines sériques de type complément.

Les surfaces de la paroi interne du récipient qui ne sont pas revêtues d'AGH, peuvent être saturées à l'aide des agents saturants classiquement utilisés dans des techniques de type phase solide ou ELISA (Enzyme Linked Immunosorbent Assay).

Par exemple, la solution d'AGH à une concentration comprise entre 1 et 10 µg/ml, peut être préparée en tampon carbonate 0,2 M pH 9,6.

Cette solution est répartie sous un volume de 75 µl dans chaque cupule d'une microplaque à fond rond de type Maxisorp U8 NUNC, puis les plaques sont incubées une nuit à 4°C.

Les cupules sont ensuite lavées à l'aide d'une solution tampon phosphate (PBS 2,5 mM pH 7,4) afin d'éliminer toutes les protéines non adsorbées directement au plastique.

Les cupules 1 sont ensuite traitées à l'aide d'une solution d'albumine à 30 g/l en tampon PBS à raison de 100 µl par cupule.

Après une incubation de 2 heures à température ambiante, les cupules sont à nouveau lavées en tampon phosphate.

### EXEMPLE 2 : Préparation d'une suspension d'érythrocytes magnétisés à l'aide de particules magnétiques ou d'une solution de ferrofluide

### A) A l'aide de particules magnétiques

On a utilisé dans cet exemple des particules paramagnétiques qui présentent une très grande homogénéité de taille (environ 300 nm de diamètre), une forte charge en matériau ferromagnétique (supérieure à 50 % en masse, environ 75 % en masse) et un état de surface plutôt hydrophobe (non fonctionnalisé). Ces particules peuvent être utilisées directement pour la magnétisation des érythrocytes, ou de préférence après un lavage préalable en détergent non ionique.

Ces particules peuvent être également utilisées après une étape de traitement avec le sérum albumine bovine (BSA) pour fixer l'érythrocyte de sorte à créer de multiples liaisons de faibles intensités entre la surface de l'érythrocyte et les particules.

A cet effet, la fixation se déroule en deux étapes, la première consiste en l'activation des particules à l'aide de la BSA et la deuxième est la mise en présence de ces particules magnétiques recouvertes de BSA avec une suspension d'érythrocytes traités ou non par des enzymes protéolytiques.

Les érythrocytes ainsi obtenues sont attirées par un champ magnétique et peuvent ainsi être utilisées dans le procédé de l'invention.

### a) Première étape : « activation » des particules ferromagnétiques à la BSA

Des particules de type Ademtech hydrophobe non fonctionnalisées de taille d'environ 300 nm, sont préalablement lavées et stockées en détergent non ionique.

Le cas échéant, ces particules sont mises en présence d'une solution d'albumine bovine à 0,1 % (poids/volume) en tampon PBS pH 7,2. Après une incubation de trente minutes à température ambiante et sous agitation non magnétique, les particules en suspension sont attirées par un aimant et le surnageant dépourvu de particules est éliminé. Le culot de particules ainsi recouvert de BSA peut être utilisé directement au cours de la phase de magnétisation des érythrocytes.

### b) Deuxième étape : Magnétisation des érythrocytes

Est ajoutée, au culot de particules ferromagnétiques obtenu à l'étape précédente, la suspension globulaire mise en tampon LISS à la concentration adéquate (possibilité d'effectuer la magnétisation des érythrocytes avec des suspensions cellulaires comprises entre 0,6 à 10 % en v/v et, le cas échéant, préalablement lavées trois fois avec de l'eau physiologique par exemple). Après avoir parfaitement homogénéisé, la suspension est incubée trente minutes à température ambiante sous agitation douce et homogène. Les érythrocytes sont ensuite lavés avec un tampon PBS pH 7,4 (deux lavages par centrifugation, trois minutes à 500 g). Le culot d'érythrocytes magnétisés peut être ensuite repris à la concentration de mise en oeuvre de l'analyse à l'aide d'un tampon type LISS (ou BFI).

Dans un exemple particulier, le rapport entre la quantité de particules mises en oeuvre et la quantité d'érythrocytes est compris entre 10 et 30 de sorte à obtenir une magnétisation efficace sans risquer de dégrader les antigènes de groupe sanguin présents à la surface de l'érythrocyte.

Les érythrocytes ainsi obtenus peuvent soit être utilisés directement en tant que réactif (pour la RAI par exemple) ou en tant qu'analyte (pour le phénotypage). A ce stade, ils peuvent également subir un traitement par des enzymes protéolytiques telles que la broméline ou la papaïne si nécessaire pour effectuer l'analyse demandée.

### B) A l'aide d'une solution de ferrofluide

### 1) Fabrication du ferrofluide prépurifié

### Matériel et méthode

### Matériel particulier et produits

- 1 filtre Stericup GS 200 ml 0,22 µm (Réf. : 107943)
- 1 aimant
- solution de NH₄OH à 28-30 % (ACROS, Réf. : 205840025)
- solution de HNO₃ à 60 % (NORMAPUR, Réf. : UN 2031)
- FeCl₂, 4H₂O (SIGMA, Réf. : 22,029-9)
- FeCl₃, 6H₂O (SIGMA, Réf. : F-2877)

### Fabrication du Ferrofuide :

### Préparation :

1/ Peser 13,51 g de FeCl₃ et dissoudre dans 20 ml d'eau déminéralisée et filtrée par agitation magnétique. Transférer la solution dans une éprouvette de 50 ml, rincer et compléter à 50 ml avec de l'eau déminéralisée filtrée (solution à 1 M).
2/ Peser 19,88 g de FeCl₂ et dissoudre dans 20 ml d'eau déminéralisée et filtrée par agitation magnétique. Transférer la solution dans une éprouvette de 50 ml, rincer et compléter à 50 ml avec de l'eau déminéralisée filtrée (solution à 2 M).
3/ Filtrer chaque solution sur filtre seringue 0,22 µm.
4/ Mettre 30 ml d'une solution d'ammoniac (NH₄OH) à 28 % dans une éprouvette de 250 ml et ajouter qsp 120 ml d'eau déminéralisée et filtrée (solution à 2 M).
5/ Peser le ballon en verre de 11 du Rotavapor à vide.

### Incubation :

6/ Mettre 10 ml de la solution de FeCl₂ (2 M) et 40 ml de la solution de FeCl₃ (1 M) dans le ballon 1 L pour Rotavapor, homogénéiser la solution en agitant le ballon à la main.
7/ Ajouter 200 ml d'eau déminéralisée filtrée, homogénéiser la solution en agitant le ballon à la main.
8/ Placer le ballon de façon inclinée sur un appareil de type Rotavapor.
9/ Faire tourner le ballon à vitesse maximum pour bien homogénéiser la solution.
10/ Ajouter 120 ml de la solution d'ammoniac 2 M.
11/ Laisser tourner le ballon pendant 15 minutes.
12/ Mettre 105 ml d'acide nitrique (HNO₃) à 60 % dans une fiole de 1 1 et ajouter qsp 1 1 d'eau déminéralisée filtrée (solution à 1 M).
13/ Après les 15 minutes de rotation, placer le ballon sur l'aimant pendant 4 minutes.
14/ Aspirer la totalité du surnageant.
15/ Resuspendre le culot.

### 1er lavage :

16/ Placer le ballon sur le Rotavapor et le faire tourner à vitesse maximum.
17/ Ajouter 200 ml de la solution d'HNO₃ à 1 M.
18/ Laisser tourner le ballon pendant 10 minutes.
19/ Resuspendre le culot.
20/ Répéter l'opération jusqu'à dissolution complète du culot.
21/ Placer le ballon sur pour faire décanter la suspension de fer.
22/ Aspirer le surnageant.
23/ Resuspendre le culot.
Les étapes 16 à 23 peuvent être répétées si nécessaire.

### Dilution finale :

26/ Peser le ballon.
27/ En déduire la masse du culot obtenu et calculer le rendement de la réaction :
   (Masse culot obtenu / Masse de fer initiale) x 100
   Masse de fer initiale = 14,77 g
   55 % < rendement
28/ Ajouter 200 ml d'eau filtrée dans le ballon.
29/ Placer le ballon sur le Rotavapor.
30/ Resuspendre le culot.
31/ Filtrer la solution sur un filtre Stericup à 0,22 µm.

### Préparation de la solution mère de ferrofluide :

Diluer le ferrofluide ainsi obtenu dans un tampon LISS ou une solution physiologique à la concentration voulue dans un flacon. Conservation du ferrofluide et/ou de la solution diluée à 4°C.

### 2) Magnétisation des Erythrocytes

### a) possibilité 1 :

- mise en contact des érythrocytes avec la solution de ferrofluide diluée entre 0,3 et 0,5 % (v/v) dans du LISS ou dans un tampon physiologique afin d'obtenir la concentration désirée en érythrocytes de la suspension finale, de préférence entre 0,5 % et 3 % en érythrocyte.

### b) possibilité 2 :

- amener la solution de ferrofluide à une DO à 450 nm équivalente à 0,9 dans du LISS,
- ajouter 10 µl de culot globulaire à 80 % d'hématocrite pour 240 µl solution de ferrofluide.

### EXEMPLE 3 : Exemple de protocole et matériels et méthodes pour la RAI

### A) Protocole

1. Prendre une microplaque 96 puits revêtue avec AGH (Anti-Globuline Humaine)
2. Déposer 50 µl de Tampon 1 (solution de gel) dans autant de cupules que nécessitera le test RAI.
3. Déposer 60 µl de solution LISS à 3 % en BSA chaque cupule utilisée.
4. Déposer 12 µl d'échantillon (sérum ou plasma) à analyser.
5. Déposer 25 µl d'hématies magnétisées de panel RAI (hématie 1 dans le puits 1, hématie 2 dans le puits 2 et hématie 3 dans le puits 3).
6. Incuber la microplaque 20 min à 37°C.
7. Sortir la microplaque de l'incubateur.
8. Mettre la plaque sur le teleshake™ (agitateur de microplaque commercialisé par H+P LAB., Allemagne) adapté pour ce protocole (voir figures 1A et 1B).
9. Agiter 5 min 30 sec. à 500 tours/min.
10. Interpréter

### Plaque coatée (revêtue d'AGH) avant et après dépôt (voir figures 2A et 2B).

### B) Interprétation (vue de dessus) (voir figures 3 et 4)

1. Un culot central, régulier et lisse sera interprété comme négatif (voir figure 3).
2. Toute ouverture du culot central sera considérée comme positif.
3. Un tapis de cellule sur l'ensemble de la surface du puits sera un positif fort (voir figure 4).

### C) Composition des éléments nécessaires à la réaction

1. Tampon 1
   1.1. Ramener la BSA 30 % à une concentration de 10 % dans du tampon LISS.
   1.2. Peser le Sephadex™ G-100 superfine (600 mg pour 20 ml de tampon 1) (Réf. 17.0061.01 - Amersham Bioscience).
   1.3. Dissoudre le Sephadex™ dans la BSA ramenée précédemment à 10 % dans le LISS.
   1.4. Laisser le tampon 1 une nuit à 4 °C avant utilisation.
2. Solution LISS à 3 % en BSA (voir composition d'une solution type LISS dans la description) ou utiliser une solution LISS du commerce.
3. Tampon LISS (voir composition dans la description).
4. Hématies magnétisées de panel RAI (voir Exemple 2).
5. Microplaque révêtue d'anti immunoglobuline.

Microplaque NUNC maxisorp de 96 puits coatée avec une antiglobuline Humaine monoclonale anti-IgG entre 1 et 5 µg /ml (voir exemple précédent pour les conditions de coating).

### EXEMPLE 4 : Résultats

### A) Etude de receveurs

Le protocole a été testé sur 308 échantillons de receveurs.

Panel d'hématies magnétisées 15 jours avant le début des analyses

**Tableau 1 : Comparaison avec une technique standard Diamed par gel centrifugation**

| RAI | | PROCEDE DE L'INVENTION | | | |
|---|---|---|---|---|---|
| | | Positifs | Douteux | Négatifs | TOTAL |
| DIAMED | Positifs | 4 | 0 | 0 | 4 |
| | Négatifs | 3 | 2 | 299 | 304 |
| TOTAL | | 7 | 2 | 299 | 308 |

Seuls 5 échantillons sont discordants sur 308, soit un taux de concordance avec Diamed de 98,4 %.

Tous les échantillons positifs sont vus par le procédé de l'invention.
Sensibilité 100 % (n=4)

Parmi les 304 négatifs, on peut noter 5 discordants soit 98,3 % de corrélation

| | |
|---|---|
| Taux de faux positifs réels : | 1 % |
| Taux de douteux : | 0,6 % |
| Spécificité (n = 304) : | 98,3 % |

### B) Etude de panels de positifs

### 1) Premier panel testé : 22 sérums positifs :

- 7 sérums avec leurs dilutions : deux échantillons de sérum présentant un anticorps anti-D, deux échantillons avec un anticorps anti-Fya, un avec un anticorps anti-Kell, un avec un anti-E et un avec un anti-S.
Résultats : aucun échantillon raté.

### 2) Deuxième panel (Life Therapeutics)

- 32 sérums

**Tableau 2**

| Anticorps | Nombre d'échantillons | Anticorps | Nombre d'échantillons | Anticorps | Nombre d'échantillons |
|---|---|---|---|---|---|
| CDE | 2 | Jkb | 3 | Lub | 1 |
| C | 3 | Fyb | 2 | E | 3 |
| D slide | 5 | Jka | 1 | Jsa | 1 |
| e | 2 | Kpb | 2 | Lua | 1 |
| Fya | 4 | S | 2 | | |

Panel testé avec le protocole de l'invention (décrit dans les exemples)
Résultats : aucun sérum positif raté.

Seulement 2 échantillons n'ont pas montré une concordance parfaite sur les 3 hématies testées en screening :
2 hématies faussement positives.

### 3) Test de receveurs du Centre Hospitalier Régional & Universitaire de Lille (CHR&U de Lille, France)

4 échantillons receveurs du CHR&U de Lille ont été détectés positifs par notre méthode et confirmés en méthode de référence DIAMED.

### 4) Limite de détection

### Protocole de l'invention

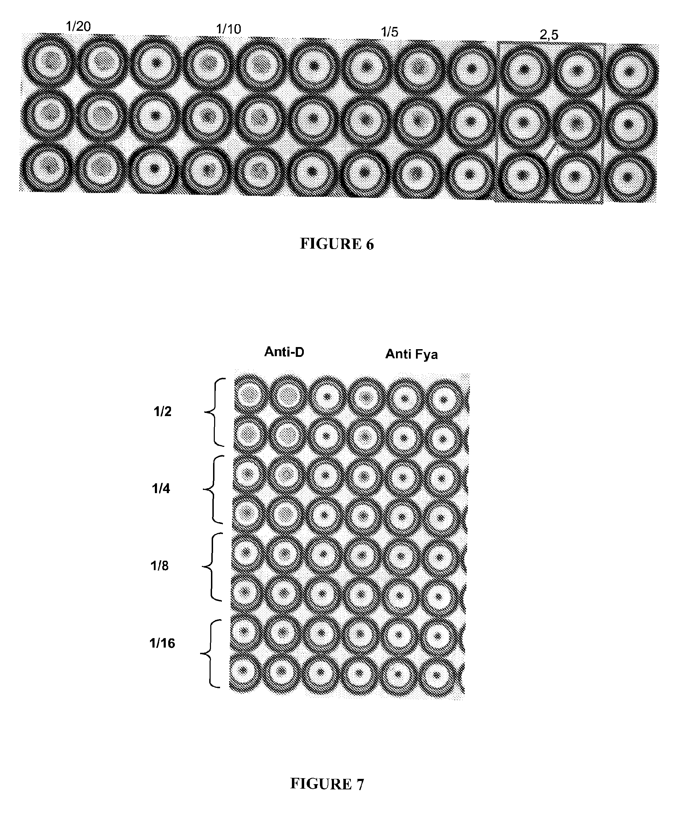

Afin de tester la limite de détection de la méthode décrite nous avons utilisé l'anti-D à 20 ng/ml du CNRGS (centre National de Référence des Groupes Sanguins) ainsi qu'un anticorps anti-Fya du contrôle Qualité, tous deux dilués dans du polycontrol.
- Anti-D CNRGS
   Dilution en polycontrol : douteux à 1 croix sur l'hématie homozygote à 2,5 ng/ml.
   Voir Figure 5
   Dilution en plasma AB : 1 croix sur l'hématie homozygote à 2,5 ng/ml.
   Voir Figure 6
- Titrage de l'anti- D et de l'anti- Fya du contrôle qualité en sérum AB
   Voir Figure 7

### 5) Répétabilité et Reproductibilité

### Protocole de l'invention

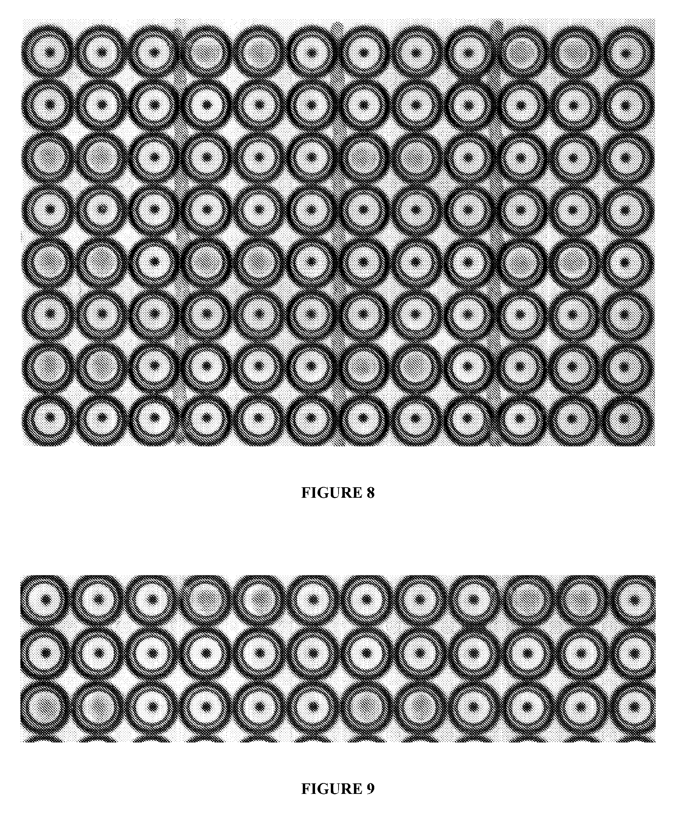

Afin de tester le protocole 8 échantillons négatifs et 4 échantillons positifs (anti-D) ont été passés sur 2 plaques différentes et 3 fois sur une même plaque.
- 3 fois sur la même plaque (test intra-plaque) (voir figure 8)
- sur une autre plaque (test inter-plaque à comparer avec la figure 8) (voir figure 9).

Les résultats observés montrent une excellente reproductibilité et répétabilité du procédé de l'invention.

### EXEMPLE 5 : Evaluation de la sensibilité de la méthode RAI selon le procédé de l'invention (appelée Qwalys ™) comparée à la technologie DiaMed™ et à la technique standard en tube BFI à l'antiglobuline (« tube LISS »)

### A) Magnétisation des hématies

Couplage par adsorption passive des hématies sur des particules magnétiques

### Procédure :

Des particules magnétiques de polystyrène non fonctionnalisées de diamètre compris en moyenne entre 500 nm et 1 µm et pouvant contenir en moyenne environ de 30 à 70 % de ferrite sont ici utilisées (type Estapor™, Merck-Chimie S.A.S./Estapor Microspheres, Fontenay-Sous-Bois, France ; ou type Ademtech, Parc Scientifique Unitec 1, 4 Allée du Doyen Georges Brus 33600 Pessac, France).

Ces particules de polystyrène peuvent contenir suivant les modèles proposés par exemple par la gamme Estapor™ ou Ademtech de 10 à 70 % de ferrite ou d'oxyde de fer et avoir des diamètres moyens compris entre 300 nm et 1 300 nm selon les modèles choisis.

Les particules magnétiques en général fournies à 10 % (p/p) sont ramenées à 1 % (10 mg/ml) avec le tampon de dilution des hématies, de préférence un tampon d'adsorption de type tampon BFI (Basse force ionique).

Les particules peuvent être préalablement lavées avec ce même tampon ou en tampon PBS si nécessaire avant dilution pour éliminer le cas échéant toute trace de détergent avant mise en contact avec les hématies.

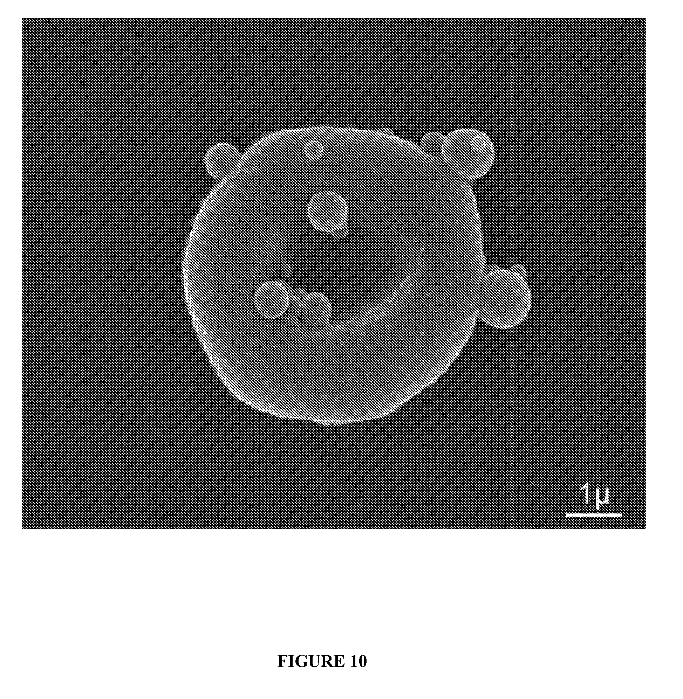

Une suspension d'hématies tests à 1 % en BFI est mise en contact volume à volume avec la suspension de particules magnétiques à 1 %, puis le mélange est agité doucement (voir figure 10 pour une photographie au microscope électronique d'une hématie magnétisée prête à l'emploi ainsi obtenue).

Bien que l'adsorption passive soit réalisée à l'essentiel au bout de quelques minutes, on peut prolonger la mise en contact jusqu'à 30 minutes, voire 1 à 2 heures à des températures allant de 4°C à 37°C, ou encore une nuit ou plus à 4°C si nécessaire avant leur utilisation.

### B) Protocole RAI sans lavages et sans centrifugagtion en cupule microplaque revêtue d'anti-globuline humaine avec des hématies magnétisées prêtes à l'empoi

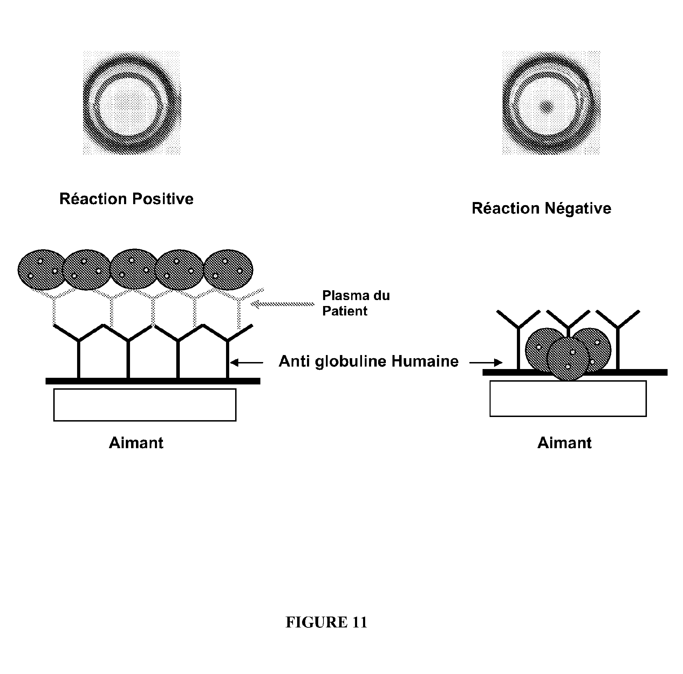

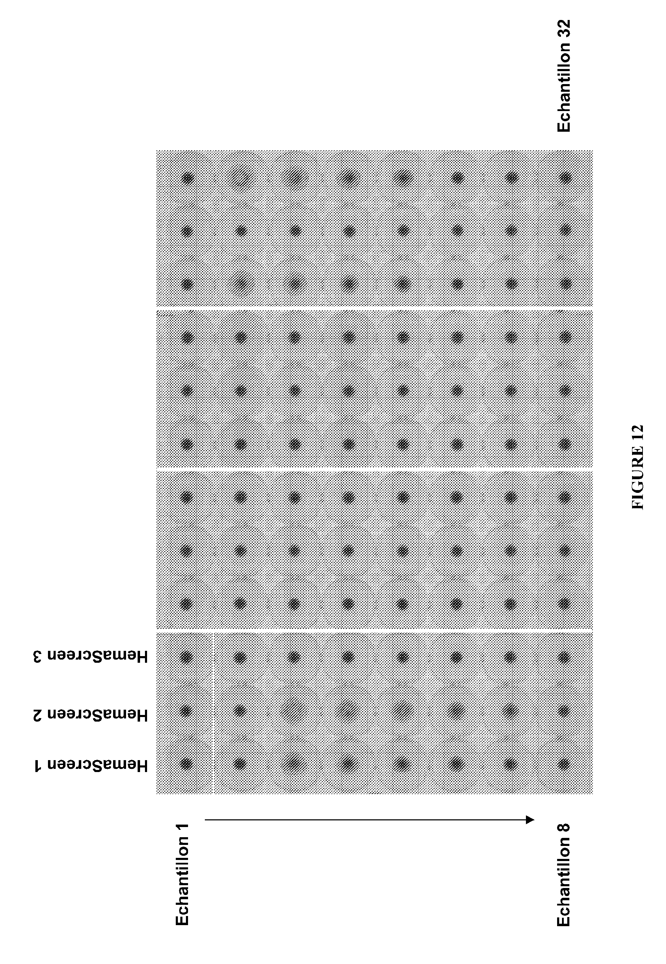

1- Ajouter 50 µl d'une solution de gel (« Tampon 1 » nommée également Nanolys™) de type Sephadex™ G-100™ superfine à 3 % dans une solution d'albumine à 10 % en BFI dans chaque cupule de microplaque en U revêtue d'antiglobuline humaine (Microplaque revêtue d'antiglobuline nommée ScreenLys™).
2- Ajouter 60 µl de solution LISS à 3 % en BSA (nommée ScreenDiluent™).
3- Ajouter 12 µl d'échantillon (sérum ou plasma) à analyser et 25 µl d'hématies magnétisées de panel RAI (nommée Hemascreen™) au-dessus de ScreenDiluent™.
4- Incuber la microplaque ScreenLys ™ ainsi obtenue 20 min à 37°C.
5- Après incubation, mettre la microplaque sur un agitateur magnétique pendant environ 5 min.
6- Lire les résultats obtenus à l'oeil ou au moyen d'un lecteur automatique (voir figures 11 et 12 pour l'interprétation et les résultats types obtenus sur un panel de 3 hématies Hemascreen™ 1, 2 et 3).

### C) Résultats

**Tableau 3 : Sensibilité comparée pour les 3 techniques exprimées en titre (dilution de 2 en 2) d'anticorps spécifiques détectés**

| Spécificité anticorps | Titre QWALYS™ | Titre DiaMed™ | Titre « tube LISS » |
|---|---|---|---|
| Anti-D | 8 | 4 | 4 |
| Anti-D | 4 | 4 | 16 |
| Anti-c | 8 | 4 | 8 |
| Anti-c | 16 | 8 | 8 |
| Anti-E | 16 | 64 | 16 |
| Anti-e | 8 | 4 | 4 |
| Anti-K | 2 | 2 | 2 |
| Anti-Fy^{a} | 16 | 8 | 4 |
| Anti-Fy^{a} | 8 | 2 | 8 |
| Anti-Jk^{a} | 8 | 16 | 8 |
| Anti-Jk^{a} | 4 | ? | 4 |
| Anti-S | 4 | 8 | 2 |
| Anti-S | 8 | 8 | 4 |

**Tableau 4 : Etude de sensibilité comparée pour les 2 techniques Qwalys™ et DiaMed™ sur un panel d'anticorps spécifiques**

| Spécificité | Nombre | QWALYS™ | DiaMed™ |
|---|---|---|---|
| Anti-D | 26 | 26 | 22 |
| Anti-E | 10 | 8 | 9 |
| Anti-c | 6 | 6 | 6 |
| Anti-e | 1 | 1 | 1 |
| Anti-K | 12 | 10 | 12 |
| Anti-Fy^{a} | 10 | 10 | 10 |
| Anti-Jk^{a} | 5 | 5 | 5 |
| Anti-Jk^{b} | 1 | 1 | 0 |
| Anti-S | 1 | 1 | 1 |
| Anti-s | 1 | 1 | 1 |
| Anti-Lu^{b} | 2 | 2 | 2 |
| Echantillons avec Mélanges anticorps | 16 | 16 | 16 |
| **Total** | **91** | **87** | **85** |
| **% positif** | | **95,6** | **93,4** |

**Tableau 5 : Etude de sensibilité comparée pour les 3 techniques Qwalys™, DiaMed™ et « tube Liss » sur un panel d'échantillons de donneurs ou anténataux**

| Nombre d'échantillons | QWALYS™ Positif | DiaMed™ Positif |
|---|---|---|
| 1590 | 32 (2,01 %) | 24 (1,51 %) |
| Nombre de positif avec « tube LISS » (à l'antiglobuline) | 16 | 16 |
| Spécificité | 98,99 % | 99,5 % |

### EXEMPLE 6 : Evaluation de la sensibilité de la méthode RAI selon le procédé de l'invention (Qwalys ™) comparée à la technologie DiaMed™ sur un échantillon test de référence internationale

Un standard international pour l'évaluation de la sensibilité de méthodologie RAI est utilisé ici.

Il s'agit d'un échantillon présentant un anticorps anti-RH1 (issu du National Institute for Biological Standards and Control, GB).

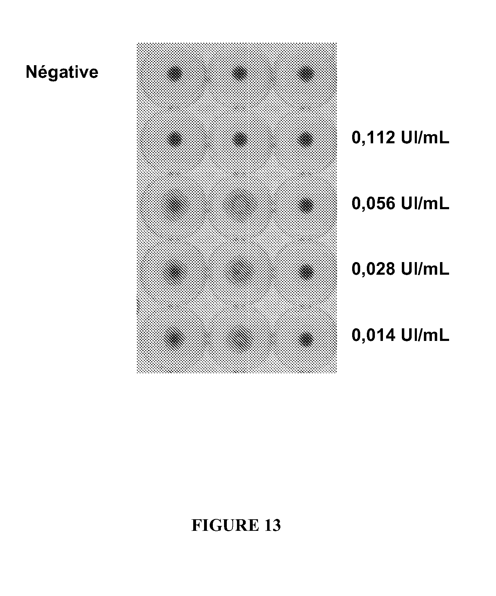

La limite de détection trouvée est proche de 0,014 UI/ml pour la technologie Qwalys™ selon l'invention utilisant les hématies magnétisées, sensibilité identique à celle trouvée pour la technique DiaMed™ (voit Figure 13).

### Conclusion

L'étude comparative de la technologie « sans lavages » et sans centrifugation Qwalys™ pour la RAI en utilisant des hématies magnétisées et une étape d'agitation en présence d'un champ magnétique (« agitation magnétique ») selon la présente invention, confirme que cette nouvelle technologie présente une spécificité et sensibilité équivalentes à celles de la technologie « gel avec centrifugation » (DiaMed™), une des techniques RAI de référence.

Un des grands avantages de la nouvelle technologie Qwalys™ selon l'invention, comparée notamment à la technologie DiaMed™ nécessitant une étape de centrifugation, est qu'elle est facilement automatisable comparée aux autres techniques car elle ne présente aucune étape de lavages (et donc de transfert d'échantillon) et de centrifugation (étape limitante pour l'automatisation complète).

## Revendications

1. Procédé de mise en évidence d'un complexe spécifique formé par réaction entre un anticorps présent dans une solution et un antigène lié à une particule magnétique, la réaction s'effectuant dans un réacteur ouvert vers le haut et à fond fermé et dont la section diminue au moins dans la zone voisine du fond pour former une paroi inclinée s'étendant jusque dans le fond, ladite paroi inclinée étant au moins en partie revêtu d'une anti-immunoglobuline ou de tout autre composé capable de se lier à l'anticorps dudit complexe formé, **caractérisé en ce qu'**il comprend les étapes suivantes :
a) préalablement à la réaction :
- le remplissage préalable du réacteur avec une substance visqueuse ou un gel homogène de manière à recouvrir au moins en partie la paroi inclinée du réacteur, ladite substance visqueuse ou ledit gel ayant une densité telle qu'elle empêche la migration des anticorps n'ayant pas formé de complexe avec les antigènes liés aux particules magnétiques vers la paroi revêtue d'anti-immunoglobuline ou de tout autre composé capable de se lier à l'anticorps lors de l'étape d)
b) la mise en contact au-dessus de la solution visqueuse contenue dans le réacteur de la solution contenant ou susceptible de contenir ledit anticorps avec la suspension de particules magnétiques portant ou susceptible de porter ledit antigène ;
c) l'incubation du réacteur pendant un temps donné nécessaire à la formation du complexe ;
d) l'application d'un champ magnétique audit réacteur et l'agitation du réacteur, de telle manière que les particules magnétiques soient entraînées vers le fond et/ou la paroi inclinée du réacteur ; et
e) la lecture à l'oeil et/ou par tout autre système de lecture appropriée, de l'image obtenue au fond du réacteur et/ou sur la paroi inclinée du réacteur revêtue de ladite anti-immunoglobuline ou de tout autre composé capable de se lier à l'anticorps, cette image obtenue permettant de mettre en évidence la présence ou non de la formation d'un complexe spécifique anticorps/antigène.

2. Procédé selon la revendication 1, **caractérisé en ce que** le mélange réactionnel obtenu par mélange entre la solution contenant ou susceptible de contenir ledit anticorps et la suspension de particules magnétiques portant ou susceptible de porter ledit antigène, contient un ou plusieurs anticorps non dirigés contre les antigènes portés par les particules magnétiques et capables de se fixer à l'anti-immunoglobuline ou à tout autre composé capable de se lier à l'anticorps.

3. Procédé selon la revendication 1 ou 2, **caractérisé en ce qu'**à l'étape d), l'application d'un champ magnétique audit réacteur et l'agitation du réacteur sont réalisées simultanément.

4. Procédé selon l'une des revendications 1 à 3, **caractérisé en ce qu'**à l'étape d), l'application du champ magnétique est réalisée au moyen d'un aimant situé à l'extérieur sous le réacteur de telle manière que les particules magnétiques soient entraînées au fond du réacteur.

5. Procédé selon l'une des revendications 1 à 4, **caractérisé en ce qu'**à l'étape d), l'agitation est réalisée au moyen d'un agitateur rotatif.

6. Procédé selon l'une des revendications 1 à 5, **caractérisé en ce qu'**à l'étape c), la durée d'incubation est comprise entre 10 min et 30 min.

7. Procédé selon l'une des revendications 1 à 6, **caractérisé en ce que** les particules magnétiques ont un diamètre compris entre 100 nm et 1,5 µm.

8. Procédé selon l'une des revendications 1 à 7, **caractérisé en ce que** les particules magnétiques contiennent au moins 40 % en poids au moins de composés ferromagnétiques.

9. Procédé selon l'une des revendications 1 à 8, **caractérisé en ce qu'**à l'étape a), le gel est un gel de dextran ou d'agarose.

10. Procédé selon l'une des revendications 1 à 9, **caractérisé en ce qu'**à l'étape b), la solution contenant ou susceptible de contenir ledit anticorps est déposée sur la solution visqueuse avant le dépôt de la suspension de particules magnétiques portant ou susceptible de porter ledit antigène.

11. Procédé selon l'une des revendications 1 à 10, **caractérisé en ce que** le réacteur est une cupule de microplaque à fond rond ou à fond V.

12. Procédé selon l'une des revendications 1 à 11, **caractérisé en ce que** la solution d'anticorps est un échantillon de plasma ou de sérum humain dans lequel on cherche à mettre en évidence la présence d'un anticorps dirigé spécifiquement contre un antigène lié à la particule magnétique et **en ce que** l'anti-immunoglobuline est une anti-immunoglobuline humaine.

13. Procédé selon l'une des revendications 1 à 12, **caractérisé en ce qu'**à l'étape e), la collecte au point le plus bas du réacteur de l'ensemble des particules magnétiques est significative de l'absence de formation de complexe spécifique anticorps/antigène ou **en ce que** la présence d'au moins une fraction visible de particules magnétiques sur la paroi inclinée du réacteur revêtue d'anti-immunoglobuline est significative de la formation dudit complexe.

14. Procédé selon l'une des revendications 1 à 13, pour la mise en évidence d'un complexe spécifique formé par réaction entre un anticorps présent dans une solution et un antigène porté par une cellule elle-même liée à une ou plusieurs particules magnétiques.

15. Procédé selon la revendication 14, pour la mise en évidence d'un complexe spécifique formé par réaction entre un anticorps anti-antigène de groupe sanguin présent dans une solution et un antigène de groupe sanguin, **caractérisé en ce que** la cellule est un érythrocyte.

16. Procédé selon la revendication 15, pour le phénotypage de globules rouges, **caractérisé en ce que** la solution d'anticorps à l'étape b) est une solution de sérum test contenant un anticorps anti-antigène de groupe sanguin connu.

17. Dispositif pour la mise en évidence d'un complexe spécifique formé par réaction entre un anticorps présent dans une solution et un antigène lié à une particule magnétique, ou pour la mise en évidence d'un complexe spécifique formé par réaction entre un anticorps anti-antigène de groupe sanguin présent dans une solution et un antigène de groupe sanguin porté par un érythrocyte lié à une ou plusieurs particules magnétiques, ou pour la RAI ou pour le phénotypage de groupe sanguin, **caractérisé en ce qu'**il comprend :
a)
- un réacteur ou un ensemble de réacteurs ouvert vers le haut et à fond fermé et dont la section diminue au moins dans la zone voisine du fond pour former une paroi inclinée s'étendant jusque dans le fond, ladite paroi inclinée étant au moins en partie revêtue d'une anti-immunoglobuline ou de tout autre composé capable de se lier à l'anticorps dudit complexe formé,
- chacun des réacteurs étant rempli en partie d'une substance visqueuse ou d'un gel, ladite substance visqueuse ou ledit gel ayant une densité telle qu'elle empêche la migration des anticorps n'ayant pas formé de complexe avec les antigènes liés aux particules magnétiques vers la paroi revêtue d'anti-immunoglobuline ou de tout autre composé capable de se lier à l'anticorps ;
b) au moins un aimant ou un ensemble d'aimants pouvant être disposé à l'extérieur sous le ou les réacteurs couplé(s) à un système d'agitation rotatif dudit ou desdits réacteurs ;
c) le cas échéant, un incubateur capable de réguler la température d'incubation des réacteurs ; et
d) le cas échéant, un lecteur capable d'évaluer la présence et la localisation des érythrocytes magnétiques à la fin de la réaction de chacun des réacteurs.

18. Kit pour la mise en évidence d'un complexe spécifique formé par réaction entre un anticorps présent dans une solution et un antigène lié à une particule magnétique, **caractérisé en ce qu'**il comprend :
a) un réactif comprenant une suspension de particules magnétiques revêtues ou destinées à être revêtues d'au moins un antigène ; et
b)
- un réacteur ou un ensemble de réacteurs ouvert vers le haut et à fond fermé et dont la section diminue au moins dans la zone voisine du fond pour former une paroi inclinée s'étendant jusque dans le fond, ladite paroi inclinée étant au moins en partie revêtue d'une anti-immunoglobuline ou de tout autre composé capable de se lier à l'anticorps dudit complexe formé,
- un container contenant une solution visqueuse ou un gel, ou le cas échéant, chacun des réacteurs étant rempli en partie de ladite substance visqueuse ou dudit gel, ladite substance visqueuse ou ledit gel ayant une densité telle qu'elle empêche la migration des anticorps n'ayant pas formé de complexe avec les antigènes liés aux particules magnétiques vers la paroi revêtue d'anti-immunoglobuline ou de tout autre composé capable de se lier à l'anticorps ; et,
c) le cas échéant, au moins un aimant ou un ensemble d'aimants pouvant être disposé à l'extérieur sous le ou les réacteurs couplé(s) à un agitateur rotatif.

19. Kit selon la revendication 18, pour la recherche d'anticorps irréguliers (RAI), **caractérisé en ce que** ledit réactif contient une suspension d'érythrocytes tests de phénotype connu sur lesquels érythrocytes sont adsorbées ou couplées lesdites particules magnétiques, ou **caractérisé en ce qu'**il comprend un second réactif contenant une suspension d'érythrocytes tests de phénotype connu destinés à être adsorbés ou couplés sur les particules magnétiques contenues dans le premier réactif.

## Patentansprüche

1. Verfahren für den Nachweis eines spezifischen und durch Reaktion eines in einer Lösung vorhandenen Antikörpers und eines mit einem Magnetpartikel verbundenen Antigens gebildeten Komplexes, wobei die Reaktion in einem nach oben offenen und bodenseitig geschlossenen Reaktor stattfindet, dessen Querschnitt sich zumindest in der Nachbarzone des Bodens verringert, um eine geneigte Wand auszubilden, die sich bis zum Boden erstreckt, und diese geneigte Wand zumindest teilweise mit einem Anti-Immunglobin oder jedweder sonstigen Verbindung beschichtet ist, die geeignet ist, sich mit dem Antikörper des gebildeten Komplexes zu verbinden, **dadurch gekennzeichnet, dass** es die nachstehenden Etappen umfasst:
a) vor der Reaktion:
- die vorherige Füllung des Reaktors mit einer zähflüssigen Substanz oder einem gleichförmigen Gel derart, dass zumindest ein Teil der geneigten Wand des Reaktors abgedeckt wird, wobei die zähflüssige Substanz oder das gleichförmige Gel eine Dichte aufweist, die die Migration der keinen Komplex mit den mit den Magnetpartikeln verbundenen Antigenen bildenden Antikörper in Richtung der mit Anti-Immunglobin oder jedweder sonstigen Verbindung, die geeignet ist, sich anlässlich dieser Etappe d) mit dem Antikörper zu verbinden, beschichteten Wand verhindert;
b) die Zusammenführung der den Antikörper aufnehmenden oder zur Aufnahme desselben geeigneten Lösung mit der das Antigen aufnehmenden oder zur Aufnahme desselben geeigneten Magnetpartikelsuspension über der im Reaktor enthaltenen Lösung;
c) die Inkubation des Reaktors während eines für die Bildung des Komplexes erforderlichen bestimmten Zeitraums;
d) die Anwendung eines Magnetfelds auf den Reaktor und das Schütteln des Reaktors derart, dass die Magnetpartikel auf den Boden und/oder die geneigte Wand des Reaktors mitgenommen werden; und
e) das Ablesen des am Reaktorboden und/oder an der geneigten und mit dem Anti-Immunglobin oder jedweder sonstigen Verbindung, die geeignet ist, sich mit dem Antikörper zu verbinden, beschichteten Wand des Reaktors entstandenen Bilds mit bloßem Auge und/oder jedwedem sonstigen geeigneten Lesesystem, wobei das erlangte Bild den Nachweis des Vorhandenseins oder Nichtvorhandenseins der Bildung eines spezifischen Antikörper/Antigen-Komplexes ermöglicht.

2. Verfahren nach Anspruch 1, **dadurch gekennzeichnet, dass** das durch die Mischung der den Antikörper aufnehmenden oder zur Aufnahme desselben geeigneten Lösung und der das Antigen aufnehmenden oder zur Aufnahme desselben geeigneten Magnetpartikelsuspension hergestellte Reaktionsgemisch einen oder mehrere Antikörper beinhaltet, die nicht gegen die von den Magnetpartikeln aufgenommenen Antigene gerichtet und geeignet sind, sich am Anti-Immunglobin oder jedweder sonstigen Verbindung, die geeignet ist, sich mit dem Antikörper zu verbinden, zu fixieren.

3. Verfahren nach Anspruch 1 oder 2, **dadurch gekennzeichnet, dass** die Anwendung eines Magnetfelds auf diesen Reaktor und das Schütteln des Reaktors in der Etappe d) zeitgleich erfolgen.

4. Verfahren nach einem der Ansprüche 1 bis 3, **dadurch gekennzeichnet, dass** die Anwendung des Magnetfelds in der Etappe d) mittels eines außenseitig unter dem Reaktor derart angeordneten Magnets erfolgt, dass die Magnetpartikel zum Reaktorboden mitgenommen werden.

5. Verfahren nach einem der Ansprüche 1 bis 4, **dadurch gekennzeichnet, dass** das Schütteln in der Etappe d) mittels einer Umlaufschüttelmaschine erfolgt.

6. Verfahren nach einem der Ansprüche 1 bis 5, **dadurch gekennzeichnet, dass** die Inkubationsdauer in der Etappe c) zwischen 10 min. und 30 min. beträgt.

7. Verfahren nach einem der Ansprüche 1 bis 6, **dadurch gekennzeichnet, dass** die Magnetpartikel einen Durchmesser zwischen 100 nm und 1,5 µm aufweisen.

8. Verfahren nach einem der Ansprüche 1 bis 7, **dadurch gekennzeichnet, dass** die Magnetpartikel zumindest einen Gewichtsanteil von zumindest 40 % ferromagnetischer Verbindungen aufweisen.

9. Verfahren nach einem der Ansprüche 1 bis 8, **dadurch gekennzeichnet, dass** das Gel in der Etappe a) ein Dextran- oder Agarosegel ist.

10. Verfahren nach einem der Ansprüche 1 bis 9, **dadurch gekennzeichnet, dass** sich die diesen Antikörper aufnehmende oder zur Aufnahme desselben geeignete Lösung in der Etappe b) vor dem Absatz der das Antigen aufnehmenden oder zur Aufnahme desselben geeigneten Magnetpartikelsuspension auf der zähflüssigen Lösung absetzt.

11. Verfahren nach einem der Ansprüche 1 bis 10, **dadurch gekennzeichnet, dass** der Reaktor eine Mikroplattenpfanne mit rundem Boden oder V-Boden ist.

12. Verfahren nach einem der Ansprüche 1 bis 11, **dadurch gekennzeichnet, dass** die Antikörperlösung eine Plasmaprobe oder die Probe eines menschlichen Serums ist, in der das Vorhandensein eines spezifisch gegen ein mit einem Magnetpartikel verbundenes Antigen gerichteten Antikörpers nachgewiesen werden soll und dadurch, dass das Anti-Immunglobin ein menschliches Anti-Immunglobin ist.

13. Verfahren nach einem der Ansprüche 1 bis 12, **dadurch gekennzeichnet, dass** die Ansammlung sämtlicher Magnetpartikel am niedrigsten Reaktorpunkt in der Etappe e) für das Nichtvorhandensein der Bildung eines spezifischen Antikörper/Antigel-Komplexes signifikant ist und dadurch, dass das Vorhandensein von zumindest einem sichtbaren Bruchteil von Magnetpartikeln an der geneigten Wand des mit Anti-Immunglobin beschichteten Reaktors für die Bildung dieses Komplexes signifikant ist.

14. Verfahren nach einem der Ansprüche 1 bis 13 für den Nachweis eines durch die Reaktion zwischen einem in einer Lösung vorhandenen Antikörper und einem von einer selbst mit einem oder mehreren Magnetpartikeln verbundenen Zelle aufgenommenen Antigen gebildeten spezifischen Komplexes.

15. Verfahren nach Anspruch 14 für den Nachweis eines durch die Reaktion zwischen einem in einer Lösung vorhandenen Blutgruppen-Anti-Antigen-Antikörper und einem Blutgruppenantigen gebildeten spezifischen Komplexes, **dadurch gekennzeichnet, dass** die Zelle ein Erythrozyt ist.

16. Verfahren nach Anspruch 15 für die Phänotypisierung von roten Blutkörpern, **dadurch gekennzeichnet, dass** die Antikörperlösung in der Etappe b) eine Testserumlösung ist, die einen Anti-Antigen-Antikörper einer bekannten Blutgruppe beinhaltet.

17. Vorrichtung für den Nachweis eines durch Reaktion zwischen einem in einer Lösung vorhandenen Antikörper und einem mit einem Magnetpartikel verbundenen Antigen gebildeten spezifischen Komplexes oder für den Nachweis eines durch Reaktion zwischen einem in einer Lösung enthaltenen Blutgruppen-Anti-Antigen-Antikörper und einem von einem mit einem oder mehreren Magnetpartikeln verbundenen Erythrozyt aufgenommenen Blutgruppenantigen gebildeten Komplexes oder für die Suche nach irregulären Antikörpern (RAI) oder die Blutgruppen-Phänotypisierung, **dadurch gekennzeichnet, dass** sie Folgendes umfasst:
a)
- einen nach oben geöffneten und bodenseitig geschlossenen Reaktor oder eine Reaktorreihe, dessen/deren Querschnitt zumindest in der benachbarten Zone des Bodens abnimmt, um eine geneigte Wand auszubilden, die sich bis in den Boden erstreckt, wobei diese geneigte Wand zumindest teilweise mit einem Anti-Immunglobin oder jedweder sonstigen Verbindung, die geeignet ist, sich mit dem Antikörper des gebildeten Komplexes zu verbinden, beschichtet ist,
- wobei jeder der Reaktoren teilweise mit einer zähflüssigen Substanz oder einem Gel gefüllt ist und diese zähflüssige Substanz oder dieses Gel eine Dichte aufweist, die die Migration der Antikörper, welche keinen Komplex mit den mit Magnetpartikeln verbundenen Antigenen bilden, in Richtung der mit Anti-Immunglobin oder jedweder sonstigen Verbindung, die geeignet ist, sich mit dem Antikörper zu verbinden, beschichteten Wand verhindert;
b) zumindest einen Magneten oder eine Magnetreihe, die außenseitig unter dem oder den Reaktoren angeordnet werden kann, welche(r) mit einer Umlaufschüttelmaschine des oder der Reaktoren verbunden ist/sind;
c) gegebenenfalls einen Inkubator, der geeignet ist, die Inkubationstemperatur der Reaktoren zu regulieren; und
d) gegebenenfalls eine Lesevorrichtung, die geeignet ist, das Vorhandensein oder die Lokalisierung der Magneterythrozyten zum Ende der Reaktion jedes Reaktors zu bewerten.

18. Kit für den Nachweis eines spezifischen Komplexes, der durch die Reaktion zwischen einem in einer Lösung enthaltenen Antikörper und einem mit einem Magnetpartikel verbundenen Antigen gebildet wird, **dadurch gekennzeichnet, dass** er Folgendes umfasst:
a) ein Reaktionsmittel, bestehend aus einer mit zumindest einem Antigen beschichteten oder zur Aufnahme einer solchen Beschichtung geeigneten Magnetpartikelsuspension; und
b)
- einen nach oben geöffneten und bodenseitig geschlossenen Reaktor oder eine Reaktorreihe, deren Querschnitt zumindest in der benachbarten Zone des Bodens abnimmt, um eine geneigte Wand auszubilden, die sich bis in den Boden erstreckt, wobei diese geneigte Wand zumindest teilweise mit einem Anti-Immunglobin oder jedweder sonstigen Verbindung, die geeignet ist, sich mit dem Antikörper des gebildeten Komplexes zu verbinden, beschichtet ist,
- einen zähflüssige Substanz oder ein Gel aufnehmenden Container, wobei jeder Reaktor teilweise mit dieser zähflüssigen Substanz oder diesem Gel gefüllt ist und diese zähflüssige Substanz oder dieses Gel eine derartige Dichte aufweist, das die Migration der Antikörper, die keinen Komplex mit den mit den Magnetpartikeln verbundenen Antigenen gebildet haben, in Richtung der mit Anti-Immunglobin oder jedweder sonstigen Verbindung, die geeignet ist, sich mit dem Antikörper zu verbunden, beschichteten Wand verhindert wird; und
c) gegebenenfalls zumindest ein Magnet oder eine Magnetreihe, das/die außenseitig unter dem oder den mit einer Umlaufschüttelmaschine gekoppelten Reaktor(en) angeordnet werden kann.

19. Kit nach Anspruch 18 für die Suche nach irregulären Antikörpern (RAI), **dadurch gekennzeichnet, dass** dieses Reaktionsmittel eine Suspension von Testerythrozyten der bekannten Phänotyps umfasst und auf den Erythrozyten die Magnetpartikel adsorbiert oder gekoppelt werden, oder dadurch, dass er ein zweites Reaktionsmittel umfasst, das eine Suspension von Testerythrozyten der bekannten Phänotyps beinhaltet, die für die Adsorption oder Kopplung an den im ersten Reaktionsmittel enthaltenen Magnetpartikeln bestimmt ist.

## Claims

1. Method for the demonstration of a specific complex formed by reacting an antibody present in a solution and an antigen bound to a magnetic particle, the reaction taking place in a reactor with an open top and sealed base whose diameter decreases at least in the area close to the base in such a way that it forms an inclined wall extending down to the base, said inclined wall being at least partially coated with an anti-immunoglobulin or any other compound capable of binding to the antibody of said formed complex, **characterised in that** it comprises the following steps:
a) Prior to the reaction,
- preliminary filling of the reactor with a viscous substance or homogeneous gel such that at least part of the inclined wall of the reactor is coated, wherein said viscous substance or said homogeneous gel has density such that it prevents the migration of antibodies which do not form complexes with the antigens bound to the magnetic particles towards the wall coated with anti-immunoglobulin or any other compound capable of binding to the antibody during step d),
b) contacting the solution containing or likely to contain said antibody with the magnetic particle suspension carrying or likely to carry said antigen at a point above the viscous solution in the reactor,
c) incubation of the reactor, preferably for the period of time required for the formation of the complex,
d) application of a magnetic field to said reactor and stirring of the reactor such that the magnetic particles are either drawn towards the base and/or inclined wall of the reactor, and
e) reading with the naked eye and/or by any other suitable reading system of the image obtained at the base of the reactor and/or inclined wall of the reactor coated with said anti-immunoglobulin or any other compound capable of binding to the antibody, the image obtained making it possible to demonstrate the presence or otherwise of the formation of a specific antibody/antigen complex.

2. Method according to claim 1, **characterised in that** the reaction mixture obtained by mixing the solution containing or likely to contain said antibody and the magnetic particle suspension carrying or likely to carry said antigen contains one or more antibodies not directed against the antigens carried by the magnetic particles and capable of binding to the anti-immunoglobulin or to any other compounds capable of binding to the antibody.

3. Method according to claim 1 or 2, **characterised in that** in step d), application of a magnetic field to said reactor and reactor stirring are carried out simultaneously.

4. Method according to one of claims 1 to 3, **characterised in that** in step d), application of a magnetic field is carried out by means of a magnet located externally below the reactor such that the magnetic particles are drawn towards the base of the reactor.

5. Method according to one of claims 1 to 4, **characterised in that** in step d), stirring is carried out by means of a rotary stirrer.

6. Method according to one of claims 1 to 5, **characterised in that** in step c), the duration of incubation is between 10 min and 30 min.

7. Method according to one of claims 1 to 6, **characterised in that** the magnetic particles have a diameter between 100 nm and 1.5 µm.

8. Method according to one of claims 1 to 7, **characterised in that** the magnetic particles contain at least 40% by weight of ferromagnetic compounds.

9. Method according to one of claims 1 to 8, **characterised in that** in step a), the gel is a dextran or agarose gel.

10. Method according to one of claims 1 to 9, **characterised in that** in step b), the solution containing or likely to contain said antibody is deposited on the viscous solution prior to depositing the magnetic particle suspension carrying or likely to carry said antigen.

11. Method according to one of claims 1 to 10, **characterised in that** the reactor is a microplate cupule with a round or V-shaped base.

12. Method according to one of claims 1 to 11, **characterised in that** the antibody solution is a human plasma or serum sample wherein demonstration of the presence of an antibody directed specifically against an antigen bound to the magnetic particle is being researched and **in that** the anti-immunoglobulin is a human anti-immunoglobulin.

13. Method according to one of claims 1 to 12, **characterised in that** in step e), collection of magnetic particles at the lowest point in the reactor is **characterised by** the absence of the formation of a specific antibody/antigen complex or in that the presence of at least a visible fraction of magnetic particles on the inclined wall of the reactor coated with the anti-immunoglobulin is characteristic of the formation of said complex.

14. Method according to one of claims 1 to 13, for demonstration of a specific complex formed by a reaction between an antibody present in a solution or an antigen carried by a cell itself bound to one or more magnetic particles

15. Method according to claim 14, for demonstration of a specific complex formed by a reaction between the anti-antigen antibody of the blood group present in a solution and an antigen of the blood group, **characterised in that** the cell is an erythrocyte.

16. Method according to claim 15, for phenotyping red cells, **characterised in that** the antibody solution in step b) is a test serum solution containing an anti-antigen antibody of a known blood group.

17. Device for the demonstration of a specific complex formed by reaction between an antibody present in a solution and an antigen bound to a magnetic particle, or for demonstration of a specific complex formed by reaction between an anti-antigen antibody of the blood group present in a solution and an antigen of the blood group carried by an erythrocyte bound to one or more magnetic particles, or for IAR or phenotyping of a blood group, **characterised in that** it includes:
a)
- a reactor or set of reactors with an open top and sealed base whose diameter decreases at least in the area close to the base in such a way that it forms an inclined wall extending down to the base, said inclined wall being at least partially coated with an anti-immunoglobulin or any other compound capable of binding to the antibody of said formed complex,
- each of the reactors being partially filled with a viscous substance or a gel, wherein said viscous substance or said gel has density such that it prevents the migration of antibodies which do not form complexes with the antigens bound to the magnetic particles towards the wall coated with anti-immunoglobulin or any other compound capable of binding to the antibody,
b) at least one magnet or set of magnets that can be arranged externally under the reactor(s) coupled to a rotary stirring system of said reactor(s),
c) if need be, an incubator capable of regulating the incubation temperature of the reactors, and
d) if need be, a reading system capable of evaluating the presence and localisation of magnetised erythrocytes at the end of the reaction in each of the reactors.

18. Kit for the demonstration of a specific complex formed by reaction between an antibody present in a solution and an antigen bound to a magnetic particle **characterised in that** it includes:
a) a reagent including a suspension of magnetic particles coated or intended to be coated with at least an antigen, and
b)
- a reactor or set of reactors with an open top and sealed base and whose diameter decreases at least in the area close to the base in order to form an inclined wall extending down to the base, said inclined wall being at least partially coated with an anti-immunoglobulin or a compound capable of binding to the antibody of said formed complex,
- a container containing a viscous solution or a gel, or if need be, each reactor being partially filled with said viscous substance or said gel, wherein said viscous substance or said gel has density such that it prevents the migration of antibodies which do not form complexes with the antigens bound to the magnetic particles towards the wall coated with anti-immunoglobulin or any other compound capable of binding to the antibody, and,
c) if need be, at least one magnet or set of magnets which can be placed externally below the reactor(s) coupled to a rotary stirrer.

19. Kit according to 18, for irregular agglutinin research (IAR), **characterised in that** said reagent contains a suspension of test erythrocytes of known phenotype to which erythrocytes are adsorbed or coupled said magnetic particles, or **characterised in that** it includes a second reagent containing a suspension of test erythrocytes of known phenotype for adsorption or coupling to the magnetic particles contained in the first reagent.
